# EUROPÄISCHE PATENTSCHRIFT

(11) **EP 2 534 166 B1**
(45) Veröffentlichungstag und Bekanntmachung des Hinweises auf die Patenterteilung: **08.04.2015**
(21) Anmeldenummer: 11702458.8
(22) Anmeldetag: 08.02.2011
(51) Int. Cl.: C07J 41/00, C07J 43/00, A61K 31/567, A61P 5/34

(54) **PROGESTERONREZEPTORANTAGONISTEN**
PROGESTERONE RECEPTOR ANTAGONISTS
ANTAGONISTES DU RÉCEPTEUR DE LA PROGESTÉRONE

(30) Priorität: 10.02.2010 DE 102010007722
(43) Veröffentlichungstag der Anmeldung: 19.12.2012
(73) Patentinhaber: Bayer Intellectual Property GmbH, 40789 Monheim (DE)
(72) Erfinder: SCHWEDE, Wolfgang, 16548 Glienicke (DE); KLAR, Ulrich, 13503 Berlin (DE); MÖLLER, Carsten, 10115 Berlin (DE); ROTGERI, Andrea, 13503 Berlin (DE); BONE, Wilhelm, 13467 Berlin (DE); HUWE, Christoph, 13503 Berlin (DE)
(86) Internationale Anmeldenummer: PCT/EP2011/051781
(87) Internationale Veröffentlichungsnummer: WO 2011/098437

(56) Entgegenhaltungen:
- FUHRMANN ET AL.: J. MED. CHEM., Bd. 43, 20. Dezember 2002 (2002-12-20), Seiten 5010-5016, XP001064233, in der Anmeldung erwähnt

## Beschreibung

Die Erfindung betrifft 17-Hydroxy-17-pentafluorethyl-estra-4,9(10)-dien-11-aryl-Derivate der Formel I mit Progesteron antagonisierender Wirkung und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von Uterusfibroiden (Myomen, uterine Leiomyome), der Endometriose, von schweren Menstruationsblutungen, Meningiomen, hormonabhängigen Mammakarzinomen und von mit der Menopause assoziierten Beschwerden oder zur Fertilitätskontrolle und Notfallkontrazeption.
Diese Verbindungen sind wertvolle pharmazeutische Wirkstoffe. Sie können unter anderem zur Herstellung pharmazeutischer Präparate zur Behandlung von Uterusfibroiden oder der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption verwendet werden. Zur Behandlung von Uterusfibroiden und der Endometriose können die erfindungsgemäßen Verbindungen auch sequentiell in Kombination mit Gestagenen verabreicht werden. In einem solchen Behandlungsregime könnten die erfindungsgemäßen Verbindungen über einen Zeitraum von 1 - 6 Monaten gegeben werden, gefolgt von einer Behandlungspause oder einer sequentiellen Behandlung mit einem Gestagen über einen Zeitraum von 2 - 6 Wochen oder gefolgt von der Behandlung mit einem oralen Kontrazeptivum (OC-Kombinationen) über den gleichen Zeitraum.
Die Wirksamkeit der erfindungsgemäßen Verbindungen als Progesteronrezeptorantagonist wurde in vitro in Transaktivierungstests und in vivo an der Ratte (Terminierung der frühen Schwangerschaft) gezeigt.
Verbindungen mit antagonistischer Wirkung am Progesteronrezeptor (kompetitive Progesteronrezeptorantagonisten) sind seit 1982 bekannt (RU 486; EP57115) und seither zahlreich beschrieben worden. Progesteronrezeptorantagonisten mit fluorierter 17α-Seitenkette wurden in WO 98/34947 und Fuhrmann et al., J. Med. Chem. 43, 5010 - 5016 (2000) veröffentlicht.

Als 4-Substituent am 11β-Phenylring ist in verschiedenen klinischen und präklinischen Progesteronrezeptor-Antagonisten die Dimethylaminofunktionalität gewählt worden (z.B. Mifepriston, Onapriston, Ulipristal, Proellex). Vorteile des basischen Stickstoffatoms ist zum Beispiel eine, besonders im sauren wässrigen Medium, erhöhte Löslichkeit. Wie mehrfach beschrieben wurde, werden durch metabolischen Abbau einer oder beider Methylgruppen am Stickstoffatom Verbindungen mit einer aromatischen NH₂-Gruppe freigesetzt. Diese Anilin-Metabolite können potentielle Nebenwirkungen aufweisen. Onapriston ist ein Progesteronrezeptorantagonist mit einer Dimethylaminofunktionalität, der sich in klinischen Studien befand. Bei einigen Patienten wurden Abnormalitäten in Leberfunktionstests beobachtet, die schließlich zur Einstellung der klinischen Studien geführt haben. Auch für Mifepriston, den einzigen auf dem Markt befindlichen ProgesteronrezeptorAntagonisten, der ebenfalls eine Dimethylaminogruppe in der 4-Position des 11β-Phenylrestes trägt, wurden erhöhte Transaminaseaktivitäten beschrieben. Für beide Verbindungen, Onapriston und Mifepriston, wurden Verbindungen mit teilweiser oder vollständiger Demethylierung als Hauptmetabolite gefunden. Kürzlich wurden publiziert, dass eine Behandlung mit Proellex in höheren Dosierungen ebenfalls zu einer Induktion von Leberenzymen führt.

Aufgabe der vorliegenden Erfindung ist es, hoch potente kompetitive Progesteronrezeptorantagonisten zur Verfügung zu stellen, die die Vorteile eines basischen Stickstoffatoms in der 4-Position des 11β-Phenylrestes aufweisen, aber keine freien Aniline als Metabolite bilden können und damit alternative Behandlungsmöglichkeiten gynäkologischer Erkrankungen zu schaffen.

Diese Aufgabe wurde gelöst, indem zwischen die Aminogruppe und den 11β-Phenylrestes eine Methylengruppe eingeführt wurde. Die Verbindungen des allgemeinen Anspruchs 1 sind daher besonders geeignet um diese Aufgabe zu lösen. Während einige der unter Anspruch 1 genannten Verbindungen eine leicht reduzierte antagonistische Potenz am Progesteronrezeptor zeigen im Vergleich zu den analogen Anilinen, wurde überraschend gefunden, dass der Einbau von Carbonylfunktionen oder Sulfonylfunktionen in einer bestimmten Entfernung zur 11β-Position des Steroidgerüstes zu einer deutlichen Erhöhung der antagonistischen Potenz führt. Einige dieser Verbindungen zeigen auch in präklinischen in vivo Versuchen eine sehr starke Progesteronrezeptor-antagonistische Aktivität und eine hervorragende Selektivität insbesondere gegenüber dem Glucocorticoidrezeptor. Diese Verbindungen sind daher bevorzugt. Besonders zu nennen sind die unter den experimentellen Beispielen 3, 11, 12, 13, 14 und 17 beschrieben Verbindungen, wobei besonders für die unter Beispiel 11 und 12 beschriebenen Verbindungen hervorragende in vivo Eigenschaften in präklinischen Tests gefunden wurden.

Die vorliegende Erfindung betrifft 17-Hydroxy-17-pentafluorethyl-estra-4,9(10)-dien-11-aryl-Derivate mit der allgemeinen chemischen Formel I: 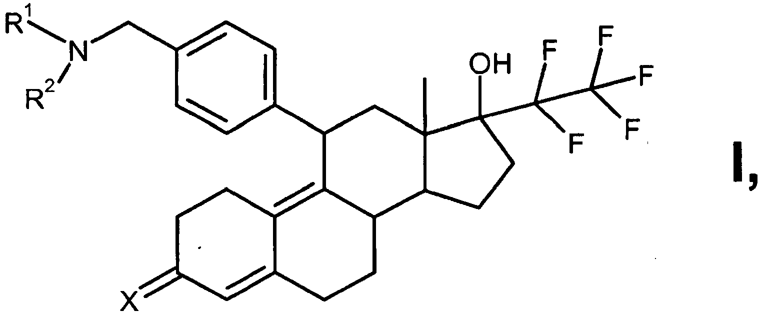 worin
- R¹ und R²: gleich oder verschieden sind und für
Wasserstoff,
einen gegebenenfalls Dimethylamin-substituierten C₁-C₁₀-Alkylrest,
einen gegebenenfalls ein-, zwei- oder mehrfach mit Halogen (-F, -Cl, -Br, -I), -OH, -O-Alkyl, -C(O)OH, -C(O)OAlkyl, -C(O)NH₂, -C(O)NHAlkyl, -C(O)NDialkyl, -C(O)NHAryl,-C(O)NHHeteroaryl, -NH₂, -NH(C₁-C₁₀-Alkyl), -N(C₁-C₁₀-Alkyl)₂, insbesondere-N(CH₃)₂, -NHC(O)Alkyl, -NO₂, -N₃, -CN, -C₁-C₁₀-Alkyl, -C₁-C₁₀-Perfluor-Alkyl, -C₁-C₁₀-Acyl, -C₁-C₁₀-Acyloxy, -SO₂NH₂ -SO₂NHAlkyl oder -SO₂NDialkyl substituierten 6-10-gliedrigen Arylrest,
einen gegebenenfalls ein-, zwei- oder mehrfach mit den oben genannten Substituenten des 6-10-gliedrigen Arylrests substituierten 5-10-gliedrigen Heteroarylrest,
einen am Arylring gegebenenfalls ein-, zwei- oder mehrfach mit den oben genannten Substituenten des 6-10-gliedrigen Arylrests substituierten C₁-C₆-Arylalkylrest oder einen am Heteroarylring gegebenenfalls ein-, zwei- oder mehrfach mit den oben genannten Substituenten des 6-10-gliedrigen Arylrests substituierten C₁-C₆-Heteroarylalkylrest stehen
oder aber
- R¹ und R²: gemeinsam Bestandteil eines gegebenenfalls am Kohlenstoff Alkyl-, Carboxyl-, Alkoxycarbonyl-" Alkylcarbonyl-, Aminocarbonyl-, Arylalkyl-, Heteroarylalkyl-, Aminoalkyl-substituierten bzw. am Stickstoff Alkyl-, Alkanoyl-, Carboxyl-, Alkoxycarbonyl-, Phenyl-, Phenylalkyl-, Pyridinyl-, Pyrimidinyl-, Pyrazinyl-, Sulfonyl-, Benzoyl-, Alkylsulfonyl-, Arylsulfonyl-, Aminocarbonyl-, Aminocarbonylalkyl, Arylalkyl-, Heterocyclylakyl-, Heteroarylalkyl- und Aminoalkyl-substituierten 3-10-gliedrigen Ringes sind, der gegebenenfalls Stickstoff-, Sauerstoff- oder Schwefelatome, die gegebenenfalls zum Sulfoxid oder Sulfon oxidiert sein können enthält, wobei an den 3-10-gliedrigen Ring gegebenenfalls ein Aromat ankondensiert sein kann,
- X: für ein Sauerstoffatom oder, NOR³ oder NNHSO₂R³,
- R³: ausgewählt ist aus der Gruppe, umfassend Wasserstoff, C₁-C₁₀-Alkyl, Aryl
und ihre Salze, Solvate oder Solvate der Salze, einschließlich aller Kristallmodifikationen.
Die erfindungsgemäßen Verbindungen der allgemeinen Formel I können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen einschließlich der Racemate. Aus solchen Mischungen von Enantiomeren und/oder Diastereomeren lassen sich die stereoisomer einheitlichen Bestandteile in bekannter Weise isolieren.
Jeder der genannten Substituenten am Steroid-Grundgerüst kann sowohl in einer α- als auch in einer β-Stellung vorliegen.
Sofern die erfindungsgemäßen Verbindungen in tautomeren Formen vorkommen können, umfasst die vorliegenden Erfindung sämtliche tautomere Formen.
Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.
Physiologisch unbedenkliche Salze der erfindungsgemäßen Verbindungen umfassen - wenn eine basische Funktion enthalten ist - Salze mit anorganischen oder organischen Säuren, insbesondere von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.
Physiologisch unbedenkliche Salze der erfindungsgemäßen Verbindungen umfassen - wenn eine saure Funktion enthaltend ist - Alkalimetallsalze, Erdalkalimetallsalze öder Ammoniumsalze, wie sie durch Umsetzung mit entsprechenden anorganischen oder organischen Basen erhalten werden können. Beispielhaft und vorzugsweise seien genannt Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclo-hexylamin, Dimethylaminoethanol, Prokain, Dibenzylamin, N-Methylmorpholin, Arginin, Lysin, Ethylendiamin, N-Methylpiperidin, N-Methyl-glukamin, D-Methyl-glukamin, Ethyl-glukamin, 1,6-Hexadiamin, Glukosamin, N-Methylglycin, 2-Amino-1,3-propandiol, Tris-hydroxy-methylaminomethan und 1-Amino-2,3,4-butantriol.
Als Solvate werden im Rahmen der Erfindung solche Formen der erfindungsgemäßen Verbindungen bezeichnet, welche in festem oder flüssigem Zustand eine Adduktbildung mit Lösungsmittelmolekülen zeigen. Das Lösungsmittel kann dabei in einem stöchiometrischen oder auch unstöchiometrischen Verhältnis vorliegen. Bei stöchiometrischen Solvaten spricht man auch von Hemi-, (Semi-), Mono-, Sesqui-, Di-, Tri-, Tetra-, Penta-, usw. Solvaten. Hydrate sind eine spezielle Form der Solvate, bei denen die Koordination mit Wasser erfolgt. Außerdem umfasst die vorliegende Erfindung auch Prodrugs der erfindungsgemäßen Verbindungen. Der Begriff "Prodrugs" umfasst Verbindungen, welche während ihrer Verweilzeit im Körper zu erfindungsgemäßen Verbindungen beispielsweise durch enzymatische oder hydrolytische Prozesse umgesetzt werden.
Im Rahmen der vorliegenden Erfindung haben die Substituenten, soweit nicht anders spezifiziert, die folgende Bedeutung:
Alkyd steht für eine gerad- oder verzweigtkettige Alkylgruppe mit 1-6 Kohlenstoffatomen, wie beispielsweise Methyl, Ethyl, Propyl, Isopropyl, n-Butyl, sec.-Butyl, Isobutyl, tert.-Butyl, n-Pentyl, Isopentyl, Neopentyl Hexyl, Heptyl, Octyl, Nonyl und Decyl.
   Der Alkylrest kann gegebenenfalls C₁-C₄-Dialkylamin-, insbesondere Dimethylaminsubstituiert sein.
Aryl steht für einen mono- bis tricyclischen aromatischen, substituierten oder unsubstituierten carbocyclischen Rest, wie zum Beispiel Phenyl oder Naphthyl.
   Der Arylrest kann ein-, zwei- oder mehrfach mit Halogen (-F, -Cl, -Br, -I), -OH, -O-Alkyl, - CO₂H, -CO₂-Alkyl, -C(O)NH₂, -C(O)NHAlkyl, -C(O)NDialkyl, -C(O)NHAryl, - C(O)NHHeteroaryl, -NH₂, -NH(C₁-C₁₀-Alkyl), -N(C₁-C₁₀-Alkyl)₂, insbesondere -N(CH₃)₂,-NHC(O)Alkyl, -NO₂, -N₃, -CN, -C₁-C₁₀-Alkyl, -C₁-C₁₀-Perfluor-Alkyl, -C₁-C₁₀-Acyl, -C₁-C₁₀-Acyloxy, -SO₂NH₂ oder -SO₂NHAlkyl, -SO₂NDialkyl substituiert sein.
Heteroaryl steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Benzofuranyl, Benzothiophenyl, Chinolinyl, Furyl, Imidazolyl, Indazolyl, Indolyl, Isochinolinyl Oxazolyl, Pyridazinyl, Pyridyl, Pyrimidyl, Pyrrolyl, Thiazolyl, Thienyl, Pyrazolyl, Isoxazolyl, Pyrazinyl, Chinolyl oder Tetrazolyl. Der Heteroarylrest kann ein-, zwei- oder mehrfach mit Halogen (-F, -Cl, -Br, -I), -OH, -O-Alkyl, -CO₂H, -CO₂-Alkyl, -C(O)NH₂, -C(O)NHalkyl, -C(O)Ndialkyl, -C(O)NHaryl,-C(O)NHHeteroaryl, -NH₂, -NH(C₁-C₁₀-Alkyl), -N(C₁-C₁₀-Alkyl)₂, insbesondere -N(CH₃)₂,-NHC(O)Alkyl, -NO₂, -N₃, -CN, -C₁-C₁₀-Alkyl, -C₁-C₁₀-Perfluor-Alkyl, -C₁-C₁₀-Acyl, -C₁-C₁₀-Acyloxy, -SO₂NH₂ oder -SO₂NHalkyl, -SO₂NDialkyl substituiert sein.
Arylalkyl steht für Arylalkylgruppen, die im Ring bis zu 14 Kohlenstoffatome, bevorzugt 6-10 Kohlenstoffatome, und in der Alkylkette 1-8, bevorzugt 1-4, Kohlenstoffatome enthalten können. Als Arylalkylreste kommen beispielsweise Benzyl, Phenylethyl, Naphthylmethyl oder Naphthylethyl in Betracht.
   Der Arylteil des Arylalkylrests kann ein-, zwei- oder mehrfach mit Halogen (-F, -Cl, -Br, -I),-OH, -O-Alkyl, -CO₂H, -CO₂-Alkyl, -C(O)NH₂, -C(O)NHAlkyl, -C(O)NDialkyl, -C(O)NHAryl,-C(O)NHHeteroaryl, -NH₂, -NH(C₁-C₁₀-Alkyl), -N(C₁-C₁₀-Alkyl)₂, insbesondere -N(CH₃)₂,-NHC(O)Alkyl, -NO₂, -N₃, -CN, -C₁-C₁₀-Alkyl, -C₁-C₁₀-Perfluor-Alkyl, -C₁-C₁₀-Acyl, -C₁-C₁₀-Acyloxy, -SO₂NH₂ oder-SO₂NHalkyl, -SO₂NDialkyl substituiert sein.
Heteroarylalkyl steht für Heteroarylalkylgruppen, wobei Heteroaryl die oben definierte Bedeutung hat und die in der Alkylkette 1-8, bevorzugt 1-4, Kohlenstoffatome enthalten können. Als Heteroarylalkylreste kommen beispielsweise Furylmethyl, Thienylethyl oder Pyridylpropyl in Betracht.
   Der Heteroarylteil des Heteroarylalkylrests kann ein-, zwei- oder mehrfach mit Halogen (-F, - Cl, -Br, -I), -OH, -O-Alkyl, -CO₂H, -CO₂-Alkyl, -C(O)NH₂, -C(O)NHAlkyl, -C(O)NDialkyl,-C(O)NHAryl, -C(O)NHHeteroaryl, -NH₂, -NH(C₁-C₁₀-Alkyl), -N(C₁-C₁₀-Alkyl)₂, insbesondere-N(CH₃)₂, -NHC(O)Alkyl, -NO₂, -N₃, -CN, -C₁-C₁₀-Alkyl, -C₁-C₁₀-Perfluor-Alkyl, -C₁-C₁₀-Acyl,-C₁-C₁₀-Acyloxy, -SO₂NH₂ oder -SO₂NHalkyl, -SO₂NDialkyl substituiert sein.
   Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt. Ganz besonders bevorzugt ist die Substitution mit einem Substituenten.
   Für Verbindungen, in denen R¹ und R² gemeinsam Bestandteil eines gegebenenfalls substituierten Ringes sind, kann dieser Ring 3-10-gliedrig sein und neben dem vorhandenen Stickstoffatom nur Kohlenstoffatome oder aber bis zu 2 weitere Heteroatome tragen. Als weitere Heteroatome sind besonders, gegebenenfalls substituierter Stickstoff, Sauerstoff, gegebenenfalls oxidierter Schwefel zu nennen. Als Substituenten am Kohlenstoff kommen Alkylgruppen, Carboxylgruppen, Alkylcarboxylgruppen, Alkylcarbonylgruppen, Aminocarbonylgruppen, Arylalkylgruppen, Heteroarylalkylgruppen, Aminoalkylgruppen in Betracht. Als Substituenten am Stickstoff kommen besonders Alkylgruppen, Alkanoylgruppen, Alkylcarboxylgruppen, Carboxylgruppen, Phenylgruppen, Phenylalkylgruppen, Pyridinyle, Pyrimidinyle, Pyrazinyle, Sulfonylgruppen, Benzoylgruppen, Alkyl- oder Arylsulfonyl, Aminocarbonyl, Arylalkyl, Heteroarylalkyl und Aminoalkyl in Frage. Schwefelatome im Ring können zum Sulfoxid oder Sulfon oxidiert sein. An den 3-10-gliedrigen Ring kann gegebenenfalls ein Aromat ankondensiert sein.
   Als Ringe, die von R¹ und R² gemeinsam gebildet werden sind besonders Piperidine, Piperazine, Morpholine, Diazepane, Thiomorpholine, Dioxidothiomorpholine, Tetrahydropyrrole zu nennen.
Heterocyclyl im Sinne der Erfindung ist ein nicht aromatisches mono- oder bicyclisches Ringsystem mit mindestens einem Heteroatom oder einer Heterogruppe. Als Heteroatome können Stickstoffatome, Sauerstoffatome und/oder Schwefelatome vorkommen. Als Heterogruppen können -S(O) -S(O)₂- vorkommen.

Heterocyclylalkyl steht für Heterocyclylalkylgruppen, wobei Heterocyclyl die oben definierte Bedeutung hat und die in der Alkylkette 1-6, bevorzugt 1-4, Kohlenstoffatome enthalten können. Als Heterocyclylalkylreste kommen beispielsweise Pyrrolidinoethyl in Betracht.

Ein monocyclischer Heterocyclylring gemäß der vorliegenden Erfindung kann 3 bis 8, bevorzugt 5 bis 8, besonders bevorzugt 5 oder 6 Ringatome aufweisen. Beispielhaft für monocyclische Heterocyclylreste mit 5 Ringatomen seien genannt: Pyrrolidinyl, Imidazolidinyl, Pyrazolidinyl, Pyrrolinyl und Tetrahydrofuranyl. Beispielhaft für monocyclische Heterocyclylreste mit 6 Ringatomen seien genannt: Piperidinyl, Piperazinyl, Morpholinyl, Tetrahydropyranyl und Thiomorpholinyl.

Ein bicyclischer Heterocyclylrest gemäß der vorliegenden Erfindung kann 5 bis 12, bevorzugt 8 bis 10 Ringatome aufweisen. Bevorzugt sind 5- bis 8-gliedrige, monocyclische gesättigte Heterocyclylreste mit bis zu zwei Heteroatomen aus der Reihe O, N und S. Besonders bevorzugt sind Morpholinyl, Piperidinyl und Pyrolidinyl.

Die in den jeweiligen Kombinationen bzw. bevorzugten Kombinationen von Resten im einzelnen angegebenen Restedefinitionen werden unabhängig von den jeweiligen angegebenen Kombinationen der Reste beliebig auch durch Restedefinitionen anderer Kombination ersetzt.
Ganz besonders bevorzugt sind Kombinationen von zwei oder mehreren der oben genannten Vorzugsbereiche.
Bevorzugt sind ebenfalls 17-Hydroxy-17-pentafluorethyl-estra-4,9(10)dien-11-aryl-Derivate mit der allgemeinen chemischen Formel II: 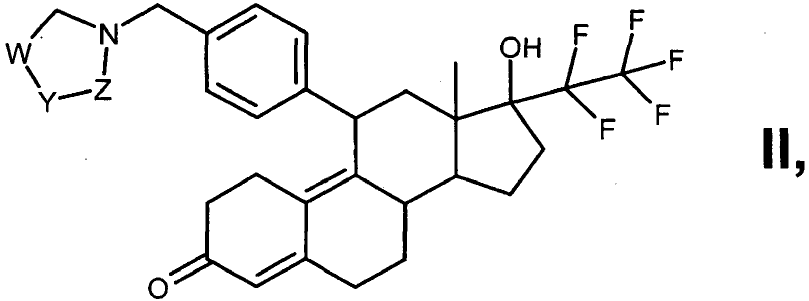 worin
- Z: -CH₂-, -CH₂-CH₂- oder -CH₂-CH₂-CH₂-,
- W: -CH₂-.
- Y: -CHR⁴-, -NR⁵-, -O- oder-SO₂- oder W und Y gemeinsam Bestandteil eines ankondensierten aromatischen Ringes sind,
- R⁴ und R⁵: Wasserstoff, -C₁-C₄-Alkyl, -(CH₂)ₘ-Phenyl, -(CH₂)ₘ-Pyridinyl, -(CH₂)ₘ-Pyrazinyl,-(CH₂)ₘ-NR⁷R⁸, -(CH₂)ₘ-C(O)-R⁶ mit m = 0, 1, 2 oder 3 oder -SO₂-C₁-C₄-Alkyl,
- R⁶: -OH, -C₁-C₄-Alkyl, -O-C₁-C₄-Alkyl, -Phenyl oder -NR⁷R⁸ und
- R⁷, R⁸: unabhängig voneinander Wasserstoff oder -C₁-C₄-Alkyl bedeuten oder gemeinsam Bestandteil eines 5- bis 7-gliedrigen Ringes sind
und ihre Salze, Solvate oder Solvate der Salze, einschließlich aller Kristallmodifikationen,
insbesondere die Verbindungen:
(11β,17β)-17-Hydroxy-11-{4-[(4-methylpiperazin-1-yl)methyl]phenyl}-17-(pentafluorethyl)estra-4,9-dien-3-on (Beispiel 2)
tert-Butyl-4-{4-[(11β,17β)-17-hydroxy-3-oxo-17-(pentafluorethyl)estra-4,9-dien-11-yl]benzyl}piperazin-1-carboxylat (Beispiel 3)
(11β,17β)-17-Hydroxy-17-(pentafluorethyl)-11-[4-(piperidin-1-ylmethyl)phenyl]estra-4,9-dien-3-on (Beispiel 4)
Methyl-1-{4-[(11β,17β)-17-hydroxy-3-oxo-17-(pentafluorethyl)estra-4,9-dien-11-yl]benzyl}piperidin-4-carboxylat (Beispiel 5)
tert-Butyl-4-{4-[(11β,17β)-17-hydroxy-3-oxo-17-(pentafluorethyl)estra-4,9-dien-11-yl]benzyl}-1,4-diazepan-1-carboxylat (Beispiel 6)
(11β,17β)-17-Hydroxy-11-[4-(morpholin-4-ylmethyl)phenyl]-17-(pentafluorethyl)estra-4,9-dien-3-on (Beispiel 7)
(11β,17β)-11-{4-[(1,1-Dioxidothiomorpholin-4-yl)methyl]phenyl}-17-hydroxy-17-(pentafluorethyl)estra-4,9-dien-3-on (Beispiel 8)
(11β,17β)-17-Hydroxy-17-(pentafluorethyl)-11-[4-(piperazin-1-ylmethyl)phenyl]estra-4,9-dien-3-on (Beispiel 10)
(11β,17β)-11-{4-[(4-Acetylpiperazin-1-yl)methyl]phenyl}-17-hydroxy-17-(pentafluorethyl)estra-4,9-dien-3-on (Beispiel 11)
(11β,17β)-17-Hydroxy-11-(4-{[4-(methylsulfonyl)piperazin-1-yl]methyl}phenyl)-17-(pentafluorethyl)estra-4,9-dien-3-on (Beispiel 12)
(11β,17β)-11-{4-[(4-Benzoylpiperazin-1-yl)methyl]phenyl}-17-hydroxy-17-(pentafluorethyl)estra-4,9-dien-3-on (Beispiel 13)
(11β,17β)-11-(4-{[4-(2,2-Dimethylpropanoyl)piperazin-1-yl]methyl}phenyl)-17-hydroxy-17-(pentafluorethyl)estra-4,9-dien-3-on (Beispiel 14)
1-{4-[(11β,17β)-17-Hydroxy-3-oxo-17-(pentafluorethyl)estra-4,9-dien-11-yl]benzyl}piperidin-4-carbonsäure (Beispiel 15)
(11β,17β)-11-[4-(1,4-Diazepan-1-ylmethyl)phenyl]-17-hydroxy-17-(pentafluorethyl)estra-4,9-dien-3-on (Beispiel 16)
(11β,17β)-11-{4-[(4-Acetyl-1,4-diazepan-1-yl)methyl]phenyl}-17-hydroxy-17-(pentafluorethyl)estra-4,9-dien-3-on (Beispiel 17)
(11β,17β)-17-Hydroxy-17-(pentafluorethyl)-11-{4-[(4-phenylpiperazin-1-yl)methyl]phenyl}estra-4,9-dien-3-on (Beispiel 25)
(11β,17β)-11-[4-(3,4-Dihydroisochinolin-2(1H)-ylmethyl)phenyl]-17-hydroxy-17-(pentafluorethyl)estra-4,9-dien-3-on (Beispiel 30)
(11β,17β)-11-{4-[(4-Benzylpiperazin-1-yl)methyl]phenyl}-17-hydroxy-17-(pentafluorethyl)estra-4,9-dien-3-on (Beispiel 31)
(11β,17β)-17-Hydroxy-17-(pentafluorethyl)-11-(4-{[4-(2-phenylethyl)piperazin-1-yl]methyl}phenyl)estra-4,9-dien-3-on (Beispiel 34)
(11β,17β)-17-Hydroxy-17-(pentafluorethyl)-11-(4-{[4-(pyrazin-2-yl)piperazin-1-yl]methyl}phenyl)estra-4,9-dien-3-on (Beispiel 35)
(11β,17β)-17-Hydroxy-17-(pentafluorethyl)-11-[4-({4-[2-(pyrrolidin-1-yl)ethyl]piperazin-1-yl}methyl)phenyl]estra-4,9-dien-3-on (Beispiel 36)
(11β,17β)-17-Hydroxy-17-(pentafluorethyl)-11-(4-{[4-(pyridin-4-ylmethyl)piperazin-1-yl]methyl}phenyl)estra-4,9-dien-3-on (Beispiel 37)
(11β,17β)-17-Hydroxy-17-(pentafluorethyl)-11-{4-[(4-phenylpiperidin-1-yl)methyl]phenyl}estra-4,9-dien-3-on (Beispiel 41)
4-{4-[(11β,17β)-17-Hydroxy-3-oxo-17-(pentafluorethyl)estra-4,9-dien-11-yl]benzyl}N,N-dimethylpiperazin-1-carboxamid (Beispiel 42)
(11β,17β)-17-Hydroxy-17-(pentafluorethyl)-11-(4-{[4-(pyridin-2-ylmethyl)piperazin-1-yl]methyl}phenyl)estra-4,9-dien-3-on (Beispiel 43)
(11β,17β)-17-Hydroxy-17-(pentafluorethyl)-11-(4-{[4-(pyridin-3-ylmethyl)piperazin-1-yl]methyl}phenyl)estra-4,9-dien-3-on (Beispiel 44)
2-(4-{4-[(11β,17β)-17-Hydroxy-3-oxo-17-(pentafluorethyl)estra-4,9-dien-11-yl]benzyl}piperazin-1-yl)-N-methylacetamid (Beispiel 45)
(11β,17β)-17-Hydroxy-17-(pentafluorethyl)-11-[4-(pyrrolidin-1-ylmethyl)phenyl]estra-4,9-dien-3-on (Beispiel 46)
(11β,17β)-17-Hydroxy-17-(pentafluorethyl)-11-(4-{[4-(pyridin-4-yl)piperazin-1-yl]methyl}phenyl)estra-4,9-dien-3-on (Beispiel 50) und
(11β,17β)-11-[4-({4-[2-(Dimethylamino)ethyl]piperazin-1-yl}methyl)phenyl]-17-hydroxy-17-(pentafluorethyl)estra-4,9-dien-3-on (Beispiel 51).
Besonders bevorzugt sind Verbindungen der Formel II, in denen entweder -C₁-C₄-Alkyl für Methyl steht und/oder in denen Z: -CH₂-CH₂- und Y: -NR⁵-, insbesondere solche, in denen zusätzlich R⁵: -C(O)-R⁶ bedeutet und ihre Salze, Solvate oder Solvate der Salze, einschließlich aller Kristallmodifikationen.

Ebenfalls bevorzugt sind die Verbindungen der allgemeinen Formel II worin
- Z: -CH₂-CH₂-oder-CH₂-CH₂-CH₂-,
- W: -CH₂-,
- Y: -CHR⁴-, -NR⁵-,
- R⁴ und R⁵: -C(O)-R⁶ oder-SO₂-C₁-C₄-Alkyl und
- R⁶: -OH, -C₁-C₄-Alkyl, -O-C₁-C₄-Alkyl bedeutet
und ihre Salze, Solvate oder Solvate der Salze, einschließlich aller Kristallmodifikationen.

Bevorzugt sind ebenfalls 17-Hydroxy-17-pentafluorethyl-estra-4,9(10)-dien-11-aryl-Derivate mit der allgemeinen chemischen Formel III: 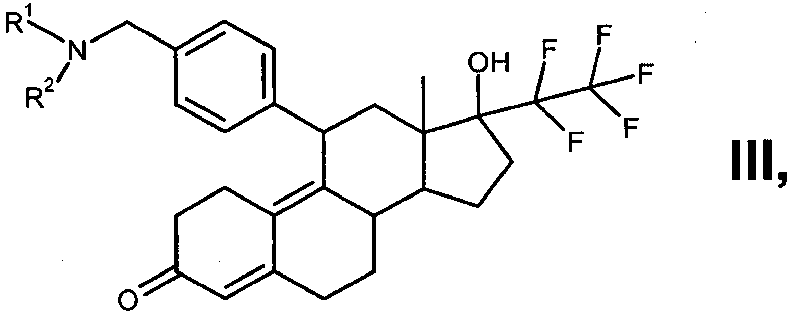 worin
- R¹ und R²: gleich oder verschieden sind und unabhängig voneinander Wasserstoff, C₁-C₄-Alkyl-, Di-C₁-C₄-Alkylamino-C₁-C₄-Alkyl-, einen gegebenenfalls Halogen-, C₁-C₄-Alkoxy-,-SO₂NH₂, -C(O)NH₂, -C(O)O-C₁-C₄-Alkyl, -NH-C(O)-C₁-C₄-Alkyl oder -C(O)-NH-Phenyl-substituierten Phenyl- oder C₁-C₄-Alkylphenylrest oder einen gegebenenfalls Halogen-, C₁-C₄-Alkoxy-, -SO₂NH₂, -C(O)NH₂, -C(O)O-C₁-C₄-Alkyl,-NH-C(O)-C₁-C₄-Alkyl oder -C(O)-NH-Phenyl-substituierten Pyridyl- oder C₁-C₄-Alkylpyridylrest bedeuten,
und ihre Salze, Solvate oder Solvate der Salze, einschließlich aller Kristallmodifikationen. Bevorzugt sind außerdem 17-Hydroxy-17-pentafluorethyl-estra-4,9(10)-dien-11-aryl-Derivate mit der allgemeinen chemischen Formel III worin
- R¹: Wasserstoff oder C₁-C₄-Alkyl und
- R²: Wasserstoff;
C₁-C₄-Alkyl,
Dimethylamino-C₁-C₄-Alkyl,
einen gegebenenfalls Halogen-, Alkoxy-, -SO₂NH₂, -C(O)NH₂, -C(O)O-C₁-C₄-Alkyl,-NH-C(O)-C₁-C₄-Alkyl oder -C(O)-NH-Phenyl-substituierten Phenylrest,
-C₁-C₄-Alkylphenyl,
Pyridyl- oder
-C₁-C₄-Alkylpyridyl
bedeutet und ihre Salze, Solvate oder Solvate der Salze, einschließlich aller Kristallmodifikationen.
Bevorzugt sind außerdem 17-Hydroxy-17-pentafluorethyl-estra-4,9(10)-dien-11-aryl-Derivate mit der allgemeinen chemischen Formel III worin R¹ Wasserstoff, Methyl oder Ethyl bedeutet, sowie deren Salze, Solvate oder Solvate der Salze, einschließlich aller Kristallmodifikationen. Bevorzugt sind außerdem 17-Hydroxy-17-pentafluorethyl-estra-4,9(10)-dien-11-aryl-Derivate mit der allgemeinen chemischen Formel III worin
- R²: -CH₃, -CH₂CH₃, -CH₂-CH₂-N(CH₃)₂, einen gegebenenfalls am Phenylring Cl-,-OCH₃, -SO₂NH₂, -C(O)NH₂, -C(O)OCH₃, -NH-C(O)CH₃ oder -C(O)-NH-Phenyl-substituierten -(CH₂)ₙ-Phenylrest oder einen -(CH₂)ₙ-Pyridyl mit n = 0, 1, 2 bedeutet
und deren Salze, Solvate oder Solvate der Salze, einschließlich aller Kristallmodifikationen. Bevorzugt sind ebenfalls 17-Hydroxy-17-pentafluorethyl-estra-4,9(10)-dien-11-aryl-Derivate mit der allgemeinen chemischen Formel III worin
- R¹: Wasserstoff, Methyl oder Ethyl und
- R²: -CH₃, -CH₂CH₃, -CH₂-CH₂-N(CH₃)₂, einen gegebenenfalls am Phenylring Cl-,-OCH₃, -SO₂NH₂. -C(O)NH₂, -CO₂CH₃, -NH-C(O)CH₃ oder -C(O)-NH-Phenyl-substituierten -(CH₂)ₙ-Phenylrest oder einen -(CH₂)ₙ-Pyridyl mit n = 0, 1, 2 bedeutet
und deren Salze, Solvate oder Solvate der Salze, einschließlich aller Kristallmodifikationen.

Bevorzugt sind ebenfalls 17-Hydroxy-17-pentafluorethyl-estra-4,9(10)-dien-11-aryl-Derivate mit der allgemeinen chemischen Formel III, nämlich:
(11β,17β)-11-{4-[(Dimethylamino)methyl]phenyl}-17-hydroxy-17-(pentafluorethyl)estra-4,9-dien-3-on (Beispiel 1)
(11β,17β)-17-Hydroxy-17-(pentafluorethyl)-11-(4-{[(2-phenylethyl)amino]methyl}phenyl)estra-4,9-dien-3-on (Beispiel 9)
4-({4-[(11β,17β)-17-Hydroxy-3-oxo-17-(pentafluorethyl)estra-4,9-dien-11-yl]benzyl}amino)benzolsulfonamid (Beispiel 18)
(11β,17β)-17-Hydroxy-17-(pentafluorethyl)-11-{4-[(pyridin-3-ylamino)methyl]phenyl}estra-4,9-dien-3-on (Beispiel 19)
(11β,17β)-11-[4-(Anilinomethyl)phenyl]-17-hydroxy-17-(pentafluorethyl)estra-4,9-dien-3-on (Beispiel 20)
(11β,17β)-17-Hydroxy-11-(4-{[(2-methoxyphenyl)amino]methyl}phenyl)-17-(pentafluorethyl)estra-4,9-dien-3-on (Beispiel 21)
(11β,17β)-17-Hydroxy-11-(4-{[(3-methoxyphenyl)amino]methyl}phenyl)-17-(pentafluorethyl)estra-4,9-dien-3-on (Beispiel 22)
(11β,17β)-11-(4-{[(4-Chlorphenyl)amino]methyl}phenyl)-17-hydroxy-17-(pentafluorethyl)estra-4,9-dien-3-on (Beispiel 23)
(11β,17β)-11-{4-[(Diethylamino)methyl]phenyl}-17-hydroxy-17-(pentafluorethyl)estra-4,9-dien-3-on (Beispiel 24)
(11β,17β)-17-Hydroxy-11-(4-{[methyl(pyridin-4-ylmethyl)amino]methyl}phenyl)-17-(pentafluorethyl)estra-4,9-dien-3-on (Beispiel 26)
(11β,17β)-11-[4-({[2-(Dimethylamino)ethyl](methyl)amino}methyl)phenyl]-17-hydroxy-17-(pentafluorethyl)estra-4,9-dien-3-on (Beispiel 27)
Methyl-2-({4-[(11β,17β)-17-hydroxy-3-oxo-17-(pentafluorethyl)estra-4,9-dien-11-yl]benzyl}amino)benzoate (Beispiel 28)
Methyl-4-({4-[(11β,17β)-17-hydroxy-3-oxo-17-(pentafluorethyl)estra-4,9-dien-11-yl]benzyl}amino)benzoate (Beispiel 29)
4-({4-[(11β,17β)-17-Hydroxy-3-oxo-17-(pentafluorethyl)estra-4,9-dien-11-yl]benzyl}amino)benzamid (Beispiel 32)
(11β,17β)-17-Hydroxy-11-(4-{[methyl(2-phenylethyl)amino]methyl}phenyl)-17-(pentafluorethyl)estra-4,9-dien-3-on (Beispiel 33)
N-[4-({4-[(11β,17β)-17-Hydroxy-3-oxo-17-(pentafluorethyl)estra4,9-dien-11-yl]benzyl}amino)phenyl]acetamid (Beispiel 38)
Methyl-3-({4-[(11β,17β)-17-hydroxy-3-oxo-17-(pentafluorethyl)estra-4,9-dien-11-yl]benzyl}amino)benzoate (Beispiel 39)
3-({4-[(11β,17β)-17-Hydroxy-3-oxo-17-(pentafluorethyl)estra-4,9-dien-11-yl]benzyl}amino)benzolsulfonamid (Beispiel 40)
(11β,17β)-17-Hydroxy-17-(pentafluorethyl)-11-(4-{[(pyridin-4-ylmethyl)amino]methyl}phenyl)estra-4,9-dien-3-on (Beispiel 47)
(11β,17β)-17-Hydroxy-11-(4-{[methyl(phenyl)amino]methyl}phenyl)-17-(pentafluorethyl)estra-4,9-dien-3-on (Beispiel 48)
2-({4-[(11β,17β)-17-Hydroxy-3-oxo-17-(pentafluorethyl)estra-4,9-dien-11-yl]benzyl}amino)benzamid (Beispiel 49)
(11β,17β)-17-Hydroxy-11-[4-({methyl[2-(pyridin-2-yl)ethyl]amino}methyl)phenyl]-17-(pentafluorethyl)estra-4,9-dien-3-on (Beispiel 52)
3-({4-[(11β,17β)-17-Hydroxy-3-oxo-17-(pentafluorethyl)estra-4,9-dien-11-yl]benzyl}amino)benzamid (Beispiel 53)
4-({4-[(11β,17β)-17-Hydroxy-3-oxo-17-(pentafluorethyl)estra-4,9-dien-11-yl]benzyl}amino)-N-phenylbenzamid (Beispiel 54).
Es wurde gefunden, dass die erfindungsgemäßen Verbindungen bzw. Derivate eine gute, das Progesteron antagonisierende Wirkung aufweisen. In mehreren klinischen Studien wurde gefunden, dass die Behandlung mit Progestronrezeptorantagonisten (Mifepriston, Asoprisnil, Proellex) zu einer signifikanten Schrumpfung von Uterusfibroiden und einer signifikanten Reduktion der mit diesen Uterusfibroiden assoziierten Symptome führen kann. Ferner wurde in klinischen Studien gezeigt, dass unter einer Behandlung mit den genannten Progesteronrezeptorantagonisten auch die von Endometriose verursachten Symptome (insbesondere Schmerzen) deutlich reduziert werden können.

Die Verbindungen der allgemeinen Formel I sowie deren physiologisch verträglichen und pharmazeutisch akzeptablen Salze können nach dem Fachmann bekannten Verfahren formuliert werden, wobei orale, einmal täglich zu verabreichende Dosisformen bevorzugt sind.

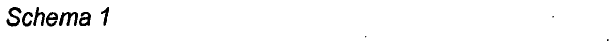

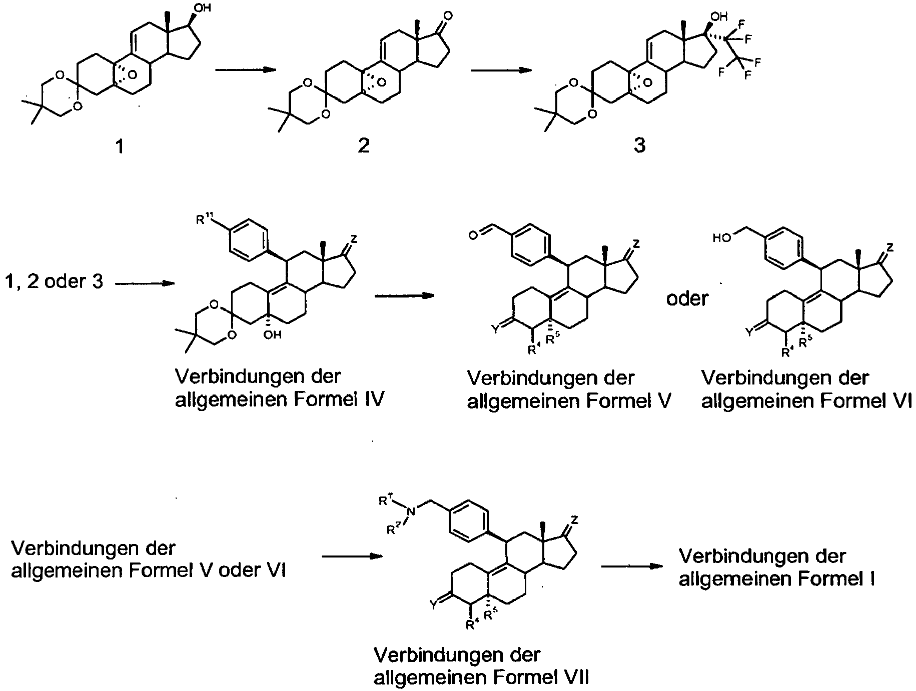

Eine Übersicht zur Herstellung von Verbindungen der allgemeinen Formel I ist in Schema 1 gezeigt. Die Darstellung der Verbindungen mit der allgemeinen chemischen Formel I erfolgt ausgehend von (5'R,8'S,10'R,13'S,14'S,17'S)-5,5,13'-Trimethyl-1',2',7',8',12',13',14',15',16',17'-decahydro-6'H-spiro[1,3-dioxan-2,3'-[5,10]epoxycyclopenta[a]phenanthren]-17'-ol (Verbindung 1, Schema 1) (Herstellung siehe Tetrahedron Lett. 26, 2069-2072 (1985) in Analogie zu den in WO 98/34947 und in WO 2008/058767 beschriebenen Verfahren. Nach Oxidation der Hydroxygruppe in Position 17 des Steroidgerüstes (Verbindung 2, Schema 1) erfolgt die Einführung der 17α-Pentafluorethylseitenkette an die entsprechenden 17-Ketoverbindungen gemäß den in WO 98/34947 und in WO 2008/058767 beschriebenen Verfahren (Verbindung 3, Schema 1). Die Einführung des 11β-Phenylsubstituenten erfolgt über konjugierte Addition von Arylgrignard- oder Aryllithiumreagenzien unter Kupfer-Katalyse. Generell können die Verbindungen 1, 2 oder 3 als Ausgangsmaterial zur Einführung des 11β-Phenylsubstituenten genutzt werden. Durch Einführung des 11β-Phenylsubstituenten werden Verbindungen der allgemeinen Formel IV erhalten, in denen Z für eine ggf geschützte Carbonylgruppe, für ein 17β-OH / 17α-H oder 17β-OH /17α-C₂F₅ steht und R¹¹ einen geschützten Aldehyd, eine Gruppe R¹²O-CH₂-oder für eine Gruppierung R^{1'},R^{2'}N-CH₂- steht. R^{1'},R^{2'}N können die bereits für Verbindungen der allgemeinen Formel I beschriebenen Bedeutungen haben, wobei funktionelle Gruppe ggf. geschützt sind. R¹² steht entweder für ein Wasserstoffatom oder eine Schutzgruppe, wobei Silylschutzgruppen bevorzugt sind. Im Falle, dass der 11β-Phenylsubstituent ausgehend von Verbindung 1 oder 2 eingeführt wird, erfolgt die Oxidation der 17-Hydroxygruppe zum Keton und die Einführung des 17α-Pentafluorethylrestes zu einem späteren Zeitpunkt.
Ausgehend von Verbindungen der allgemeinen Formel IV, in denen R¹¹ für einen geschützten Aldehyd steht, wird nach dem Fachmann bekannten Methoden die Aldehydschutzgruppe gespalten, so dass Verbindungen der allgemeinen Formel V erhalten werden, in denen Z die bereits genannten Bedeutungen haben kann. Die Spaltung der Aldehydschutzgruppe kann unter Reaktionsbedingungen erfolgen, unter denen auch die Ketalschutzgruppe an Position 3 der Steroidgerüstes zum Keton gespalten wird, aber auch unter mild sauren Bedingungen unter denen die Schutzgruppe an der Position 3 erhalten bleibt. Ausgehend von Verbindungen der allgemeinen Formel IV, in denen R¹¹ für eine Gruppe R¹²O-CH₂- steht, wird eine ggf vorhandene Alkoholschutzgruppe gespalten, so dass Verbindungen der allgemeinen Formel VI erhalten werden. Verbindungen der allgemeinen Formel V oder VI werden dann zur Einführung der Aminofunktion genutzt. Ausgehend von Aldehyden der allgemeinen Formel V erfolgt die Einführung über reduktive Aminierungen. Die hierfür eingesetzten Amine können primär oder sekundär sein und ggf Schutzgruppen tragen. Erfolgt die Einführung der Aminogruppe aufgehend von Alkoholen der allgemeinen Formel IV, so wird die Hydroxygruppe zunächst in eine geeignete Fluchtgruppe überführt und diese dann durch Umsetzung mit den entsprechenden Aminen substituiert. Als Fluchtgruppen kommen neben Chlor, Brom oder lod besonders Tosylate oder Mesylate in Frage.
Aus den Verbindungen der allgemeinen Formel VII können dann Verbindungen der allgemeinen Formel I hergestellt werden. Hierzu werden ggf vorhandene Schutzgruppen gespalten sowie die Reste R¹, und R^{2'} ggf weiter modifiziert. Als Umsetzungen der Reste R^{1'} und R^{2'} sind besonders Oxidationen oder Reduktionen, Veresterungen, Verseifungen, Alkylierung, Acylierungen von freien Valenzen am Stickstoff sowie Bildungen von Sulfonamiden zu nennen.

Als Ketalschutz- oder Acetalschutzgruppen sind beispielsweise die Ethylendioxy- oder die 2,2-Dimethylpropylen-1,2-dioxygruppe zu nennen. Hydroxygruppen werden beispielsweise in Form von Methoxymethyl, Methoxyethyl-, Tetrahydropyranyl-, Benzyl-, oder Silylethern geschützt.
Bei der Spaltung des 3-Ketals zur 3-Ketogruppe des Steroidgerüstes wird eine gegebenenfalls noch vorhandene 5α-Hydroxygruppe elimininiert, so dass Verbindungen der allgemeinen Formel I entstehen.
Soweit die Herstellung der Ausgangsverbindungen hier nicht beschrieben ist, sind diese dem Fachmann bekannt oder analog zu bekannten Verbindungen oder hier beschriebenen Verfahren herstellbar. Die Isomerengemische können nach üblichen Methoden, wie beispielsweise Kristallisation, Chromatographie oder Salzbildung, in die einzelnen Verbindungen aufgetrennt werden. Die Herstellung der Salze erfolgt in üblicher Weise, indem man eine Lösung der Verbindungen mit der allgemeinen chemischen Formel I mit der äquivalenten Menge oder einem Überschuss einer Base oder Säure, die sich gegebenenfalls in Lösung befindet, versetzt, gegebenenfalls den Niederschlag abtrennt oder in üblicher Weise die Lösung aufarbeitet.
Die resultierenden Verbindungen der Formel (I) werden gegebenenfalls mit den entsprechenden (i) Lösungsmitteln und/oder (ii) Basen oder Säuren zu ihren Solvaten, Salzen und/oder Solvaten der Salze umsetzt.
Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.
Die erfindungsgemäßen Verbindungen zeigen ein nicht vorhersehbares, wertvolles pharmakologisches, pharmakokinetisches und pharmakodynamisches Wirkprofil.
Sie eignen sich daher zur Verwendung als Arzneimittel zur Behandlung und/oder Prophylaxe von Krankheiten bei Menschen und Tieren.
Die pharmazeutische Wirksamkeit der erfindungsgemäßen Verbindungen lässt sich durch ihre Wirkung als Progesteronrezeptorantagonisten, also ihre antagonisierende Wirkung am Progesteronrezeptor erklären.
Weiterer Gegenstand der vorliegenden Erfindung ist der Einsatz der erfindungsgemäßen Verbindungen zur Behandlung und/oder Prophylaxe von Erkrankungen basierend auf hormonabhängigen hyperproliferativen Prozessen, vorzugsweise von gynäkologischen Krankheiten, insbesondere von Uterusfibroiden, der Endometriose oder von hormonabhängigen Mammakarzinomen.
Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.
Weiterer Gegenstand der vorliegenden Erfindung sind die erfindungsgemäßen Verbindungen zur Verwendung in einem Verfahren zur Behandlung und/oder Prophylaxe von Uterusfibroiden, der Endometriose und von hormonabhängigen Mammakarzinomen.
Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen. Weiterer Gegenstand der vorliegenden Erfindung ist ein Verfahren zur Behandlung und/oder Prophylaxe von Erkrankungen, insbesondere der zuvor genannten Erkrankungen, unter Verwendung von 0,1-100 mg der erfindungsgemäßen Verbindungen pro Tag und Patientin bei der Behandlung von Uterusfibroiden oder der Endometriose und für die kontrazeptive Anwendung bzw. von 0,1-500 mg der erfindungsgemäßen Verbindungen pro Tag und Patientin bei Tumor-Erkrankungen (z.B. Menginiome oder hormonabhängige Tumore wie z.B. das Mammakarzinom) und bei der Notfallkontrazeption.
Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen.
Zur Behandlung von Tumor-Erkrankungen können z.B. die folgenden Wirkstoffe/Wirkstoffklassen entweder gleichzeitig oder sequentiell verabreicht werden: SERMs, SERDs, Antiöstrogene, Aromataseinhibitoren, Kinaseinhibitoren, Angiogeneseinhibitoren und/oder Cytostatika.
Zur Behandlung von Uterusfibroiden oder der Endometriose können die erfindungemäßen Verbindungen gleichzeitig oder sequentiell mit Gestagenen oder Kombinationen aus Östrogenen und Gestagenen kombiniert werden.
In WO 96/15794 (Spicer et al., Balance Pharm. Inc.), WO 96/03130 (Stöckemann et al., Schering AG) und PCT/EP2009/003249 (Möller et al., Bayer Schering Pharma AG) sind Progesteronerezeptorantagonisten/Gestagen-Regime offenbart. Für die Behandlung von Uterusfibroiden und der Endometriose gut geeignet sind - sich gegebenenfalls wiederholende - Regime in denen der Progesteronrezeptorantagonist über einen Zeitraum von zwei bis vier Monaten gegeben wird, gefolgt von der Gabe des Gestagens über einen Zeitraum von ein bis vier Wochen. Besonders gut geeignet ist die - sich gegebenenfalls wiederholende - 84tägige Gabe des Progesteronrezeptorantagonisten gefolgt von der 14tägigen Gabe des Gestagens.
Zur Behandlung von mit der Menopause assoziierten Beschwerden kommt eine gleichzeitige oder sequentielle Verabreichung der erfindungsgemäßen Verbindungen z.B. mit SERMs, SERDs und Östrogenen in Betracht.
SERMs (Selective Estrogen Receptor Modulators) sind solche Verbindungen, die gewebeseletiv entweder eine antiestrogene bzw. estrogene Wirkung haben, beispielsweise am Uterus die Wirkung des Östrogens inhibieren, am Knochen aber eine neutrale oder dem Östrogen ähnliche Wirkung haben. Beispiele sind Clomifen, Raloxifen, Tamoxifen, Torimifen, Bazedoxifen, Lasofoxifen und Ormeloxifen.
Selektive Estrogenrezeptordestabilisatoren (SERD) sind Arzneistoffe, die den Estrogenrezeptor vollständig antagonisieren (,reine Antiöstrogene' ohne östrogene Wirkkomponente) und zu einem Abbau des Rezeptors führen (beispielsweise Fulvestrant, ZK-703 und ZK-253 ( Hoffmann J et al., J Natl Cancer Inst 2004, 96:210-218) sowie in WO 98/007740, WO 99/33855 und WO 03/045972 beschriebene Verbindungen.
Antiöstrogene sind Verbindungen die den Estrogenrezeptor vollständig antagonisieren, beispielsweise Fulvestrant.
Aromataseinhibitoren inhibieren das Enzym Aromatase und somit die Aromatisierung von Androgenen in Estrogene. Dazu gehören u.a. Anastrozole, Letrozole, Exemestane, Vorozole, Formestane and Fadrozole.
Kinaseinhibitoren sind Enzyme, die einen Phosphatrest von ATP auf andere Substrate, dort insbesondere auf Hydroxygruppen, übertragen, z.B. Sorafenib (Nexavar) oder Imatinib (Gleevec).
Angiogenesehemmer, z.B. Avastin, reduzieren bzw. blockieren die Gefäßversorgung und damit die Durchblutung eines Tumors.
Zytostatika, z.B. cis-Platin, Taxol, Taxotere sind natürliche oder synthetische Substanzen, die das Zellwachstum bzw. die Zellteilung hemmen.

Als Gestagene werden im Sinne vorliegender Erfindung entweder das natürliche Progesteron selbst verstanden oder synthetische Derivate, die wie das Progesteron selbst an den Progesteronrezeptor binden und in Dosierungen, die über der Ovulationshemmdosis liegen, die Ovulation hemmen. Als Beispiele für die synthetischen Derivate seien das Drospirenon, Gestoden, Levonorgestrel, Cyproteronacetat, Desogestrel und 3-Ketodesogestrel, Norethisteron, Norethisteronacetat und das Dienogest genannt.

Bei Kombinationen aus Gestagenen und Östrogenen handelt es sich um die Wirkstoffkombinationen, die in den an sich bekannten oralen Kontrazeptiva, beispielsweise Yasmin, Femovan, Triquilar, Marvelon, YAZ etc., enthalten sind.
Die erfindungsgemäßen Verbindungen können systemisch und/oder lokal wirken. Zu diesem Zweck können sie auf geeignete Weise, wie z.B. oral, intrauterin[är], intravaginal, parenteral, pulmonal, nasal, sublingual, lingual, buccal, rectal, dermal, transdermal, conjunctival, otisch oder als Implantat bzw. Stent appliziert werden.
Intrauterin[är] bedeutet dabei insbesondere die Applikation mittels IUS (intrauterine system) oder IUD (intrauterine device). Die intravaginale Applikation kann u.a. mittels IVR/VRS (Intra-Vaginalring/Vaginalringsystem) erfolgen.
Intrauterine oder intravaginale Applikationsformen (vergl. z.B. WO 01/47490, insbesondere Seite 1, Zeile 10 bis Seite 5, Zeile 13 und Seite 7, Zeile 19 bis Seite 58, Zeile 6, oder für Vaginalringe: WO 06/010097, insbesondere Seite 10, Zeile 22 bis Seite 14, Zeile 28) können dabei die erfindungsgemäßen Verbindungen und Nonsilikon- und/oder Silikonpolymere, insbesondere auch siloxanebasierte Elastomere (vergl. WO 01/47490, insbesondere Seite 7, Zeile 19 - Seite 15, Zeile 15), enthalten.
Für diese Applikationswege können die erfindungsgemäßen Verbindungen in geeigneten Applikationsformen verabreicht werden.
Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.
Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.
Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.
Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.
Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.
Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.
Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

Die folgenden Beispiele dienen der Erläuterung der Erfindung ohne diese in irgend einer Weise zu beschränken.

### Beispiel 1:

### (11β,17β)-11-{4-[(Dimethylamino)-methyl]phenyl}-17-hydroxy-17-(pentafluorethyl)estra-4,9-dien-3-on

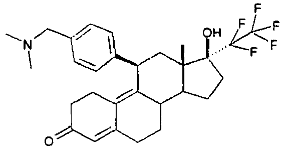

### a) (5'R,8'S,10'R,13'S,14'S,17'S)-5,5,13'-Trimethyl-17'-(pentafluorethyl)-1',2',7',8',12',13',14',15',16',17'-decahydro-6'H-spiro[1,3-dioxan-2,3'-[5,10]epoxycyclopenta[a]phenanthren]-17'-ol

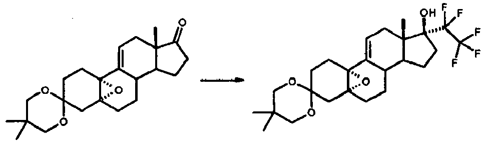

Zu 116 g kondensiertem Pentafluoriodethan in 500 ml absolutem Toluol wurden bei -70°C 50 g (5'R,8'S,10'R,13'S,14'S)-5,5,13'-Trimethyl-1',2',6',7',8',12',13',14',15',16'-decahydro-17'H-spiro[1,3-dioxan-2,3'-[5,10]epoxycyclopenta[a]phenanthren]-17'-on (Herstellung siehe Tetrahedron Lett. 26, 2069-2072 (1985) gegeben. Dazu gab man 290 ml einer 1,5 molaren Lösung von Methyllithium-Lithiumbromid-Komplex in Diethylether bei gleicher Temperatur. Anschließend wurde eine Stunde bei 0°C nachgerührt. Dann wurde das Reaktionsgemisch auf gesättigte wässrige Ammoniumchloridlösung gegeben und mit Ethylacetat extrahiert. Die organische Phase wurde mit gesättigter wässriger Natriumchloridlösung gewaschen, über Natriumsulfat getrocknet und im Vakuum eingeengt. Das Rohprodukt wurde in 200 ml Aceton aufgelöst und mit 450 ml Wasser versetzt. Das ausgefallene Produkt wurde abfiltriert und im Vakuum getrocknet.
Ausbeute 61,6 g
¹H-NMR (400 MHz, CDCl₃): δ= 6,04 brd (1 H); 3,60 d (1 H); 3,35-3,50 m (3H); 2,51 dbr (1 H); 1,06 s (3H); 0,93 s (3H); 0,85 s (3H).

### 1b) (5R,8S,11R,13S,14S,17S)-11-[4-(Dimethoxymethyl)phenyl]-5',5',13-trimethyl-17-(pentafluorethyl)-1,2,6,7,8,11,12,13,14,15,16,17-dodecahydrospiro[cyclopenta[a]phenanthren-3,2'-[1,3]dioxan]-5,17(4H)-diol

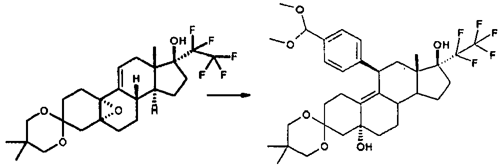

1,48 g Magnesium-Späne wurden in 5 ml THF suspendiert und unter Rühren mit 50 µl Dibromethan versetzt. Zur Suspension wurde bei 40°C eine Lösung von 10,18 ml 1-Brom-4-(dimethoxymethyl)benzol in 70 ml THF und danach für eine Stunde bei 50°C nachgerührt. Anschließend wurde die entstandene Lösung auf 0 °C gekühlt. Man addierte 40 mg CuCl und ließ weitere 15 Minuten bei 0°C nachrühren. Danach wurde eine Lösung von 5 g der unter Beispiel 1a) beschriebenen Substanz in 50 ml THF addiert. Dann ließ man das Reaktionsgemisch unter Rühren über ca. 3 Stunden auf 23°C kommen und rührte anschließend bei dieser Temperatur 10 Stunden nach. Anschließend wurde gesättigte wässrige NH₄Cl-Lösung zum Reaktionsgemisch unter externer Kühlung addiert. Man rührte weitere 30 Minuten nach und extrahierte dann mehrfach mit Ethylacetat. Die vereinigten organischen Phasen wurden mit gesättigter Natriumchloridlösung gewaschen und über Natriumsulfat getrocknet. Das Rohprodukt wurde durch Chromatographie an Kieselgel aufgereinigt. Man erhielt 6,4 g der Titelverbindung.
¹H-NMR (400 MHz, CDCl₃): δ= 7,32 d (2H); 7,21 d (2H); 5,36 s (1 H); 4,43 s (1 H); 4,32 dbr (1 H); 3,39-3,58 m (4H); 3,31 s (6H); 1,03 s (3H); 0,86 s (3H); 0,51 s (3H).

### c) 4-[(11β,17β)-17-Hydroxy-3-oxo-17-(pentafluorethyl)estra-4,9-dien-11-yl]benzaldehyd

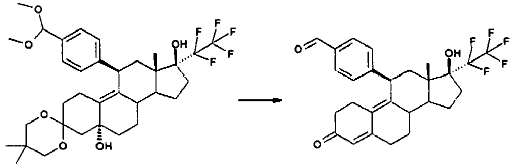

3,5 g der unter 1b) beschriebenen Verbindung wurden in 55 ml 70%iger Essigsäure gelöst. Man ließ 16 Stunden bei 30°C nachrühren. Anschließend wurde das Reaktionsgemisch auf Wasser gegossen. Man ließ weitere 5 Stunden nachrühren. Danach wurde filtriert. Der Rückstand wurde mit Wasser gewaschen, getrocknet und durch Chromatographie an Kieselgel gereinigt. Man erhielt 2,2 g der Titelverbindung.
¹H-NMR (300 MHz, CDCl₃): δ= 9,99 s (1 H); 7,80 d (2H); 7,37 d (2H); 5,80 sbr (1H); 4,51 dbr (1H); 0,58 s (3H).

### d) (11β,17β)-11-{4-[(Dimethylamino)-methyl]phenyl}-17-hydroxy-17-(pentafluorethyl)estra-4,9-dien-3-on

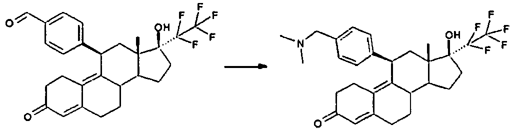

Zu einer Lösung von 200 mg der unter 1 c) beschriebenen Verbindung in 3 ml Dichlormethan wurden 0,4 ml einer 2 molaren Lösung von Dimethylamin in THF gegeben. Man ließ 15 Minuten bei 23°C nachrühren und addierte dann 171 mg Natriumtriacetoxyborhydrid. Anschließend ließ man 20 Stunden bei 23°C nachrühren. Danach wurde das Reaktionsgemisch auf gesättigte wässrige Natriumhydrogencarbonatlösung gegossen. Man extrahierte mehrmals mit Dichlormethan. Die vereinigten organischen Phasen wurden mit gesättigter wässriger Natriumchloridlösung gewaschen, über Natriumsulfat getrocknet und im Vakuum eingeengt. Das Rohprodukt wurde durch Chromatographie an Kieselgel gereinigt. Man erhielt 131 mg der Titelverbindung.
¹H-NMR (400 MHz, CDCl₃): δ= 7,30 d (2H); 7,11 d (2H); 5,78 sbr (1 H); 4,44 dbr (1 H); 3,32-3,44 m (2H); 2,75 m (1 H); 0,58 s (3H).

Die Beispiele 2-9 wurden in Analogie zu Beispiel 1 aus der unter 1c) beschriebenen Verbindung und dem jeweiligen Amin synthetisiert:

| Bsp | Struktur | Name | ¹H-NMR |
|---|---|---|---|
| 2 | 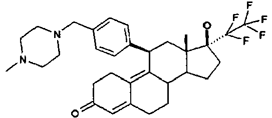 | (11β,17β)-17-Hydroxy-11-{4-[(4-methylpiperazin-1-yl)methyl]phenyl}-17-(pentafluorethyl)estra-4,9-dien-3-on | ¹H-NMR (300 MHz, CDCl₃): δ= 7,30 d (2H); 7,10 d (2H); 5,77 sbr (1H); 4,44 dbr (1H); 3,46 m (2H); 2,73 m (1H); 2,27 s (3H); 0,59 s (3H). |
| 3 | 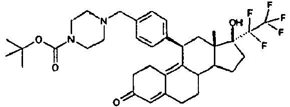 | tert-Butyl-4-{4-[(11β,17β)-17-hydroxy-3-oxo-17-(pentafluorethyl)estra-4,9-dien-11-yl]benzyl}piperazin-1-carboxylat | ¹H-NMR (300 MHz, CDCl₃): δ= 7,20 d (2H); 7,11 d (2H); 5,77 sbr (1H); 4,44 dbr (1H); 3,35-3,47 m (10 H); 1,46 s (9H); 0,58 s (3H). |
| 4 | 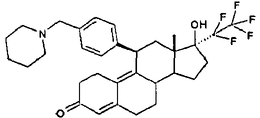 | (11β,17β)-17-Hydroxy-17-(pentafluorethyl)-11-[4-(piperidin-1-ylmethyl)phenyl]estra-4,9-dien-3-on | ¹H-NMR (400 MHz, CDCl₃): δ= 7,21 d (2H); 7,09 d (2H); 5,78 sbr (1H); 4,44 dbr (1H); 3,42 m (2H); 2,73 m (1 H); 0,59 s (3H). |
| 5 | 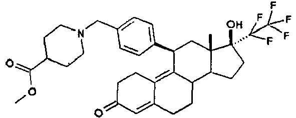 | Methyl-1-{4-[(11β,17β)-17-hydroxy-3-oxo-17-(pentafluorethyl)estra-4,9-dien-11-yl]benzyl}piperidin-4-carboxylat | ¹H-NMR (400 MHz, CDCl₃): δ= 7,18 d (2H); 7,10 d (2H); 5,78 sbr (1H); 4,45 dbr (1H); 3,67 m (5H); 3,43 m (2H); 2,70-2,90 m (4H); 0,60 s (3H). |
| 6 | 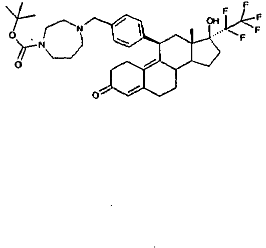 | tert-Butyl-4-{4-[(11β)-17-hydroxy-3-oxo-17-(pentafluorethyl)estra-4,9-dien-11-yl]benzyl}-1,4-diazepan-1-carboxylat | ¹H-NMR (400 MHz, CDCl₃): δ= 7,21 d (2H); 7,10 d (2H); 5,78 sbr (1H); 4,44 dbr (1H); 3,58 m (2H); 3,38-3,53 m (5H); 2,73 m (1H); 2,50-2,68 m (8H); 1,48 s (9H); 0,58 s (3H). |
| 7 | 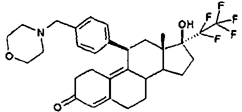 | (1β,17β)-17-Hydroxy-11-[4-(morpholin-4-ylmethyl)phenyl]-17-(pentafluorethyl)estra-4,9-dien-3-on | ¹H-NMR (300 MHz, CDCl₃): δ= 7,21 d (2H); 7,11 d (2H); 5,77 sbr (1H); 4,44 dbr (1H); 3,62-3,76 m (4H); 3,45 m (2H); 2,72 m (1H); 2,48-2,68 m (3H); 0,59 s (3H). |
| 8 | 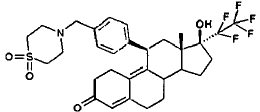 | (11β,17β)-11-{4-[(1,1-Dioxidothiomorpholin-4-yl)methyl]phenyl}-17-hydroxy-17-(pentafluorethyl)estra-4,9-dien-3-on | ¹H-NMR (300 MHz, CDCl₃): δ= 7,20 d (2H); 7,13 d (2H); 5,78 sbr (1H); 4,45 dbr (1H); 3,61 m (2H); 3,05 m (4H); 2,96 m (4H); 2,73 m (1H); 0,59 s (3H). |
| 9 | 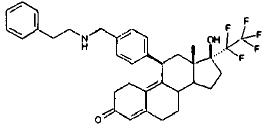 | (11β,17β)-17-Hydroxy-17-(pentafluorethyl)-11-(4-{[(2-phenylethyl)amino]methyl}phe nyl)estra-4,9-dien-3-on | ¹H-NMR (400 MHz, CDCl₃): δ= 7,27 d (2H); 7,18 m (5H); 7,09 d (2H); 5,78 sbr (1H); 4,43 dbr (1H); 3,76 m (2H); 2,92 m (2H); 2,83 m (2H); 2,71 m (1H); 0,58 s (3H). |

### Beispiel 10: (11β,17β)-17-Hydroxy-17-(pentafluorethyl)-11-[4-(piperazin-1-yl-methyl)phenyl]estra-4,9-dien-3-on

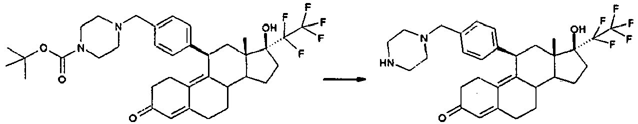

Zu einer Lösung von 180 mg der unter Beispiel 3 beschriebenen Verbindung in 3 ml Dichlormethan wurden 0,5 ml Trifluoressigsäure addiert. Man ließ 40 Minuten bei 23°C nachrühren und goss dann das Reaktionsgemisch auf gesättigte wässrige Natriumhydrogencarbonatlösung. Anschließend ließ man weitere 30 Minuten nachrühren und extrahierte anschließend mehrfach mit Dichlormethan. Die vereinigten organischen Phasen wurden mit gesättigter wässriger Natriumchloridlösung gewaschen, über Natriumsulfat getrocknet und im Vakuum eingeengt. Das Rohprodukt wurde durch Chromatographie an Kieselgel gereinigt. Man erhielt 87 mg der Titelverbindung.
¹H-NMR (400 MHz, CDCl₃): δ= 7,20 d (2H); 7,10 d (2H); 5,77 sbr (1 H); 4,43 dbr (1H); 3,38-3,52 m (2H); 2,80-2,92 m (4H); 2,72 m (1 H); 0,58 s (3H).

### Beispiel 11: (11β,17β)-11-{4-[(4-Acetylpiperazin-1-yl)methyl]phenyl}-17-hydroxy-17-(pentafluorethyl)estra-4,9-dien-3-on

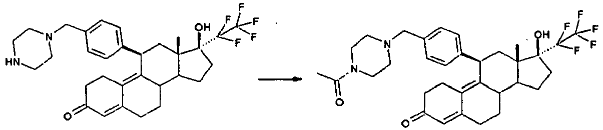

Zu einer Lösung von 80 mg der unter Beispiel 10 beschriebenen Verbindung in 2,5 ml Dichlormethan wurden 0,4 ml Triethylamin addiert. Man kühlte auf 0°C und addierte 14 µl Essigsäureanhydrid. Anschließend ließ man auf 23°C kommen und rührte 1 Stunde nach. Dann wurde das Reaktionsgemisch auf gesättigte wässrige Natriumhydrogencarbonatlösung gegossen. Man ließ weitere 30 Minuten nachrühren und extrahierte anschließend mehrfach mit Dichlormethan. Die vereingten organischen Phasen wurden mit gesättigter wässriger Natriumchloridlösung gewaschen, über Natriumsulfat getrocknet und im Vakuum eingeengt. Das Rohprodukt wurde durch Chromatographie an Kieselgel gereinigt. Man erhielt 80 mg der Titelverbindung.
¹H-NMR (400 MHz, CDCl₃): δ= 7,20 d (2H); 7,12 d (2H); 5,78 sbr (1H); 4,45 dbr (1H); 3,38-3,50 m (4H); 2,75 m (1 H); 2,06 s (3H); 0,59 s (3H).

### Beispiel 12: (11β,17β)-17-Hydroxy-11-(4-{[4-(methylsulfonyl)piperazin-1-yl]methyl}phenyl)-17-(pentafluorethyl)estra-4,9-dien-3-on

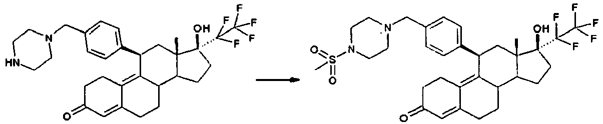

Zu einer Lösung von 96 mg der Unter Beispiel 10 beschriebenen Verbindung in 3 ml THF wurden 71 µl Triethylamin addiert. Man kühlte auf 0°C und addierte 20 µl Methansulfonsäurechlorid. Anschließend ließ man auf 23°C kommen und rührte 1 Stunde nach. Dann wurde das Reaktionsgemisch auf gesättigte wässrige Natriumhydrogencarbonatlösung gegossen. Man ließ weitere 30 Minuten nachrühren und extrahierte anschließend mehrfach mit Dichlormethan. Die vereinigten organischen Phasen wurden mit gesättigter wässriger Natriumchloridlösung gewaschen, über Natriumsulfat getrocknet und im Vakuum eingeengt. Das Rohprodukt wurde durch Chromatographie an Kieselgel gereinigt. Man erhielt 82 mg der Titelverbindung.
¹H-NMR (300 MHz, CDCl₃): δ= 7,20 d (2H); 7,13 d (2H); 5,79 sbr (1H); 4,45 dbr (1H); 3,50 m (2H); 3,15-3,35 m (4H); 2,78 s (3H); 0,59 s (3H).

### Beispiel 13: (11β,17β)-11-{4-[(4-Benzoylpiperazin-1-yl)methyl]phenyl}-17-hydroxy-17-(pentafluorethyl)estra-4,9-dien-3-on

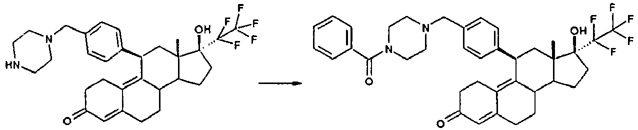

Zu einer Lösung von 300 mg der unter Beispiel 10 beschriebenen Verbindung in 10 ml THF wurden 0,22 ml Triethylamin addiert. Man kühlte auf 0°C und addierte 93 µl Benzoylchlorid. Anschließend ließ man auf 23°C kommen und rührte 1 Stunde nach. Dann wurde das Reaktionsgemisch auf gesättigte wässrige Natriumhydrogencarbonatlösung gegossen. Man ließ weitere 30 Minuten nachrühren und extrahierte anschließend mehrfach mit Dichlormethan. Die vereinigten organischen Phasen wurden mit gesättigter wässriger Natriumchloridlösung gewaschen, über Natriumsulfat getrocknet und im Vakuum eingeengt. Das Rohprodukt wurde durch Chromatographie an Kieselgel gereinigt. Man erhielt 290 mg der Titelverbindung.
¹H-NMR (300 MHz, CDCl₃): δ= 7,33-7,47 m (5H); 7,20 d (2H); 7,11 d (2H); 5,77 sbr (1 H); 4,45 dbr (1H); 3,68-3,92 m (2H); 3,49 m (2H); 3,34-3,52 m (2H); 2,73 m (1H); 0,58 s (3H).

### Beispiel 14: (11β,17β)-11-(4-{[4-(2,2-Dimethylpropanoyl)piperazin-1-yl]methyl}phenyl)-17-hydroxy-17-(pentafluorethyl)estra-4,9-dien-3-on

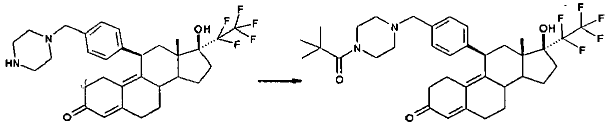

Zu einer Lösung von 300 mg der unter Beispiel 10 beschriebenen Verbindung in 10 ml THF wurden 0,22 ml Triethylamin addiert. Man kühlte auf 0°C und addierte 98 µl Pivaloylchlorid. Anschließend ließ man auf 23°C kommen und rührte 1 Stunde nach. Dann wurde das Reaktionsgemisch auf gesättigte wässrige Natriumhydrogencarbonatlösung gegossen. Man ließ weitere 30 Minuten nachrühren und extrahierte anschließend mehrfach mit Dichlormethan. Die vereinigten organischen Phasen wurden mit gesättigter wässriger Natriumchloridlösung gewaschen, über Natriumsulfat getrocknet und im Vakuum eingeengt. Das Rohprodukt wurde durch Chromatographie an Kieselgel gereinigt. Man erhielt 241 mg der Titelverbindung.
¹H-NMR (300 MHz, CDCl₃): ö= 7,21 d (2H); 7,11 d (2H); 5,78 sbr (1H); 4,43 dbr (1H); 3,55-3,70 m (4H); 3,46 m (2H); 2,74 m (1 H); 1,25 s (9H); 0,59 s (3H).

### Beispiel 15: 1-{4-[(11β,17β)-17-Hydroxy-3-oxo-17-(pentafluorethyl)estra-4,9-dien-11-yl]benzyl}piperidin-4-carbonsäure

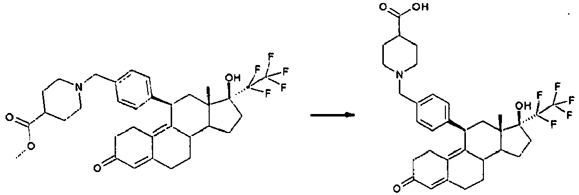

Zu einer Lösung von 200 mg der unter Beispiel 5 beschriebenen Verbindung in 5 ml Methanol 0,52 ml einer 2 molaren wässrigen Natriumhydroxidlösung addiert. Man ließ 48 Stunden bei 23°C nachrühren und verdünnte dann das Reaktionsgemisch mit 5 ml Wasser. Anschließend wurde auf 0°C gekühlt und durch Zugabe von 0,15 ml 2 normaler Salzsäure angesäuert. Das ausgefallene Produkt wurde abfiltriert, getrocknet und durch Chromatographie an Kieselgel gereinigt. Man erhielt 50 mg der Titelverbindung.
¹H-NMR (400 MHz, CDCl₃): ö= 7,25 d (2H); 7,14 d (2H); 5,74 sbr (1 H); 4,40 dbr (1 H); 3,83 d (2H); 3,70 d (2H); 2,90-3,21 m (2H); 2,70 m (1H); 1,25 s (9H); 0,58 s (3H).

### Beispiel 16: (11β,17β)-11-[4-(1,4-Diazepan-1-ylmethyl)phenyl]-17-hydroxy-17-(pentafluorethyl)estra-4,9-dien-3-on

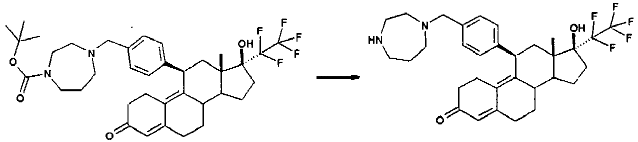

Analog zu Beispiel 10 wurden aus 170 mg der unter Beispiel 6 beschriebenen Verbindung durch Reaktion mit Trifluoressigsäure in Dichlormethan 63 mg der Titelverbindung erhalten.
¹H-NMR (400 MHz, CDCl₃): δ= 7,22 d (2H); 7,10 d (2H); 5,78 sbr (1 H); 4,43 dbr (1 H); 3,60 m (2H); 0,57 s (3H).

### Beispiel 17: (11β,17β)-11-{4-[(4-Acetyl-1,4-diazepan-1-yl)methyl]phenyl}-17-hydroxy-17-(pentafluorethyl)estra-4,9-dien-3-on

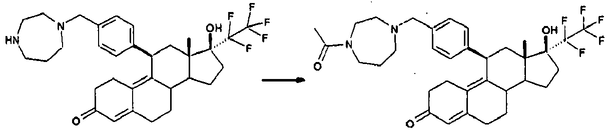

Analog zu Beispiel 11 wurdne aus 45 mg der unter Beispiel 16 beschriebenen Verbindung durch Umsetzung mit Essigsäureanhydrid in Dichlormethan 27 mg der Titelverbindung hergestellt.
¹H-NMR (300 MHz, CDCl₃): δ= 7,21 d (2H); 7,11 d (2H); 5,79 sbr (1H); 4,42 dbr (1H); 3,55-3,70 m (4H); 3,42-3,55 m (2H); 2,75 m (1 H); 2,10 s (3H); 0,57 s (3H).
Die Beispiele 18-54 wurden gemäß der folgenden Vorschrift durch Parallelsynthese hergestellt:
0,6 ml einer 0,67 molaren Suspension von Natriumtriacetoxyborhydrid in 1,2-Dichlorethan wurden vorgelegt. Man addierte 0,4 ml einer 0,5 molaren Lösung der unter 1c) beschriebenen Verbindung in 1,2-Dichlorethan und danach 0,5 ml einer 0,5 molaren Lösung des jeweiligen Amins in THF. Anschließend ließ man 12 Stunden bei 50°C nachrühren. Danach wurde das Reaktionsgemisch mit 2,5 ml Ethylacetat und 1,5 ml einer 10%igen wässrigen Natriumhydroxidlösung versetzt. Die organische Phase wurde abgetrennt und eingeengt. Die Rohprodukte wurden durch HPLC gereinigt und per HPLC-MS analysiert (Waters Acquity Ultra Performance LC, Photodiodenarraydetektor-Wellenlänge 210-350 nm, Säule Acquity UPLC BEH C18 1,7 µm, 2,1x50 mm, Säulentemperatur 60 °C, Gradient 1-99% Acetonitril in 0.1% Ameisensäure/Wasser, Flußrate 0,8 ml/min., Laufzeit 2 min.).

| Bsp. | Struktur | Name | HPLC-MS (MH⁺, RT) |
|---|---|---|---|
| 18 | 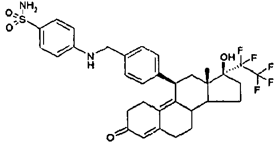 | 4-({4-[(11β,17β)-17-Hydroxy-3-oxo-17-(pentafluorethyl)estra-4,9-dien-11-yl]benzyl}amino)benzolsulfonamid | 652, 1.30 min. |
| 19 | 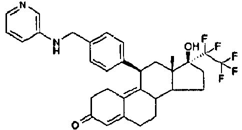 | (11β,17β)-17-Hydroxy-17-(pentafluorethyl)-11-{4-[(pyridin-3-ylamino)methyl]phenyl}estra-4,9-dien-3-on | 574, 1.04 min. |
| 20 | 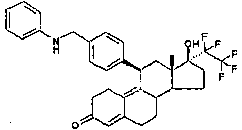 | (11β,17β)-11-[4-(Anilinomethyl)phenyl]-17-hydroxy-17-(pentafluorethyl)estra-4,9-dien-3-on | 573, 1.48 min. |
| 21 | 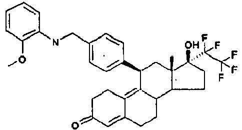 | (11β,17β)-17-Hydroxy-11-(4-{[(2-methoxyphenyl)amino]methyl}phenyl)-17-(pentafluorethyl)estra-4,9-dien-3-on | 603, 1.51 min. |
| 22 | 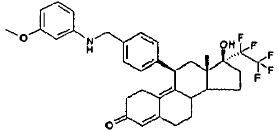 | (11β,17β)-17-Hydroxy-11-(4-{[(3-methoxyphenyl)amino]methyl}phenyl)-17-(pentafluorethyl)estra-4,9-dien-3-on | 603, 1.47 min. |
| 23 | 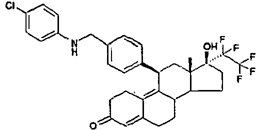 | (11β,17β)-11-(4-{[(4-Chlorphenyl)amino]methyl}phenyl)-17-hydroxy-17-(pentafluorethyl)estra-4,9-dien-3-on | 607, 1.55 min. |
| 24 | 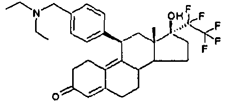 | (11β,17β)-11-{4-[(Diethylamino)methyl]phenyl}-17-hydroxy-17-(pentafluorethyl)estra-4,9-dien-3-on | 553, 1.02 min. |
| 25 | 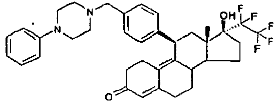 | (11β,17β)-17-Hydroxy-17-(pentafluorethyl)-11-{4-[(4-phenylpiperazin-1-yl)methyl]phenyl}estra-4,9-dien-3-on | 642, 1.10 min. |
| 26 | 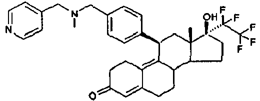 | (11β,17β)-17-Hydroxy-11-(4-{[methyl(pyridin-4-ylmethyl)amino]methyl}phenyl)-17-(pentafluorethyl)estra-4,9-dien-3-on | 602, 0.99 min. |
| 27 | 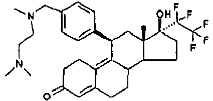 | (11β,17β)-11-[4-({[2-(Dimethylamino)ethyl](methyl)amino}methyl)phenyl]-17-hydroxy-17-(pentafluorethyl)estra-4,9-dien-3-on | 582, 0.89 min. |
| 28 | 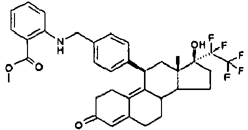 | Methyl-2-({4-[(11β,17β)-17-hydroxy-3-oxo-17-(pentafluorethyl)estra-4,9-dien-11-yl]benzyl}amino)benzoat | 631, 1.59 min. |
| 29 | 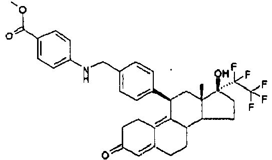 | Methyl-4-({4-[(11β,17β)-17-hydroxy-3-oxo-17-(pentafluorethyl)estra-4,9-dien-11-yl]benzyl}amino)benzoat | 631, 1.46 min. |
| 30 | 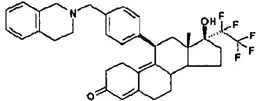 | (11β,17β)-11-[4-(3,4-Dihydroisochinolin-2(1H)-ylmethyl)phenyl]-17-hydroxy-17-(pentafluorethyl)estra-4,9-d ien-3-on | 613, 1.07 min. |
| 31 | 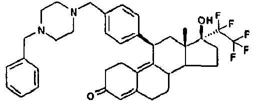 | (11β,17β)-11-{4-[(4-Benzylpiperazin-1-yl)methyl]phenyl}-17-hydroxy-17-(pentafluorethyl)estra-4,9-dien-3-on | 656, 1.09 min. |
| 32 | 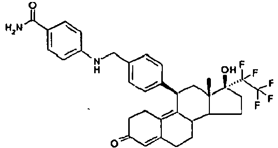 | 4-({4-[(11β,17β)-17-Hydroxy-3-oxo-17-(pentafluorethyl)estra-4,9-dien-11-yl]benzyl}amino)benzamid | 616, 1.26 min. |
| 33 | 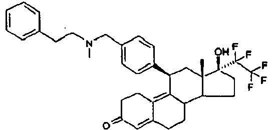 | (11β,17β)-17-Hydroxy-11-(4-{[methyl(2-phenylethyl)amino]methyl}phenyl)-17-(pentafluorethyl)estra-4,9-dien-3-on | 615, 1.09 min. |
| 34 | 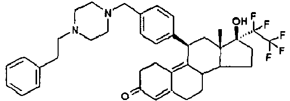 | (11β,17β)-17-Hydroxy-17-(pentafluorethyl)-11-(4-{[4-(2-phenylethyl)piperazin-1-yl]methyl}phenyl)estra-4,9-dien-3-on | 670, 1.10 min. |
| 35 | 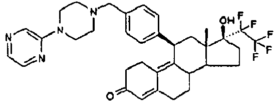 | (11β,17β)-17-Hydroxy-17-(pentafluorethyl)-11-(4-{[4-(pyrazin-2-yl)piperazin-1-yl]methyl}phenyl)estra-4,9-dien-3-on | 644, 1.02 min. |
| 36 | 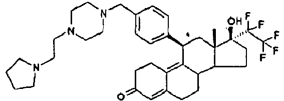 | (11β,17β)-17-Hydroxy-17-(pentafluorethyl)-11-[4-({4-[2-(pyrrolidin-1-yl)ethyl]piperazin-1-yl}methyl)phenyl]estra-4,9-dien-3-on | 663, 0.86 min. |
| 37 | 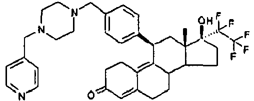 | (11β,17β)-17-Hydroxy-17-(pentafluorethyl)-11-(4-{[4-(pyridin-4-ylmethyl)piperazin-1-yl]methyl}phenyl)estra-4,9-dien-3-on | 657, 0.88 min. |
| 38 | 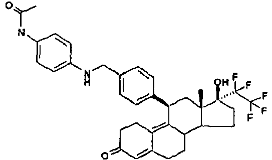 | N-[4-({4-[(11β,17β)-17-Hydroxy-3-oxo-17-(pentafluorethyl)estra-4,9-dien-11-yl]benzyl}amino)phenyl]acetamid | 630, 1.26 min. |
| 39 | 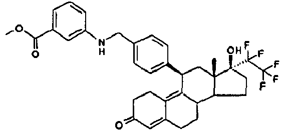 | Methyl-3-({4-[(11β,17β)-17-hydroxy-3-oxo-17-(pentafluorethyl)estra-4,9-dien-11-yl]benzyl}amino)benzoat | 631, 1.48 min. |
| 40 | 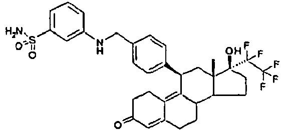 | 3-({4-[(11β,17β)-17-Hydroxy-3-oxo-17-(pentafluorethyl)estra-4,9-dien-11-yl]benzyl}amino)benzolsulfonamid | 652, 1.31 min. |
| 41 | 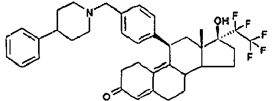 | (11β,17β)-17-Hydroxy-17-(pentafluorethyl)-11-{4-[(4-phenylpiperidin-1-yl)methyl]phenyl}estra-4,9-dien-3-on | 641, 1.11 min. |
| 42 | 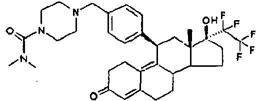 | 4-{4-[(11β,17β)-17-Hydroxy-3-oxo-17-(pentafluorethyl)estra-4,9-dien-11-yl]benzyl}-N,N-dimethylpiperazin-1-carboxamid | 637, 1.00 min. |
| 43 | 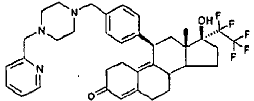 | (11β,17β)-17-Hydroxy-17-(pentafluorethyl)-11-(4-{[4-(pyridin-2-ylmethyl)piperazin-1-yl]methyl}phenyl)estra-4,9-dien-3-on | 657, 0.94 min. |
| 44 | 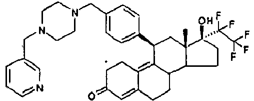 | (11β,17β)-17-Hydroxy-17-(pentafluorethyl)-11-(4-{[4-(pyridin-3-ylmethyl)piperazin-1-yl]methyl}phenyl)estra-4,9-dien-3-on | 657, 0.91 min. |
| 45 | 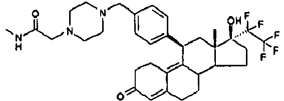 | 2-(4-{4-[(11β,17β)-17-Hydroxy-3-oxo-17-(pentafluorethyl)estra-4,9-dien-11-yl]benzyl}piperazin-1-yl)-N-methylacetamid | 637, 0.97 min. |
| 46 | 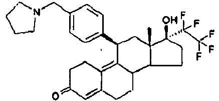 | (11β,17β)-17-Hydroxy-17-(pentafluorethyl)-11-[4-(pyrrolidin-1-ylmethyl)phenyl]estra-4,9-dien-3-on | 551, 1.02 min. |
| 47 | 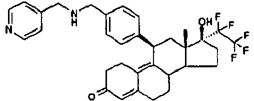 | (11β,17β)-17-Hydroxy-17-(pentafluorethyl)-11-(4-{[(pyridin-4-ylmethyl)amino]methyl}phenyl)estra-4,9-dien-3-on | 588, 0.96 min. |
| 48 | 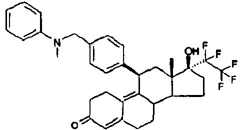 | (11β,17β)-17-Hydroxy-11-(4-{[methyl(phenyl)amino]methyl}phenyl)-17-(pentafluorethyl)estra-4,9-dien-3-on | 587, 1.55 min. |
| 49 | 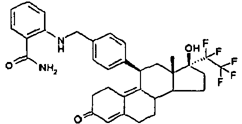 | 2-({4-[(11β,17β)-17-Hydroxy-3-oxo-17-(pentafluorethyl)estra-4,9-dien-11-yl]benzyl}amino)benzamid | 616, 1.38 min. |
| 50 | 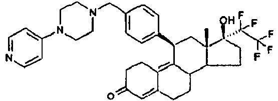 | (11β,17β)-17-Hydroxy-17-(pentafluorethyl)-11-(4-{[4-(pyridin-4-yl)piperazin-1-yl]methyl}phenyl)estra-4,9-dien-3-on | 643, 0.87 min. |
| 51 | 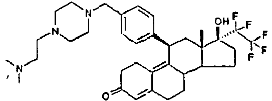 | (11β,17β)-11-[4-({4-[2-(Dimethylamino)ethyl]piperazin-1-yl}methyl)phenyl]-17-hydroxy-17-(pentafluorethyl)estra-4,9-dien-3-on | 637, 0.85min. |
| 52 | 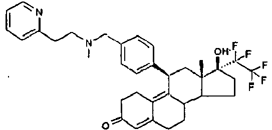 | (11β,17β)-17-Hydroxy-11-[4-({methyl[2-(pyridin-2-yl)ethyl]amino}methyl)phenyl]-17-(pentafluorethyl)estra-4,9-dien-3-on | 616, 1.04 min. |
| 53 | 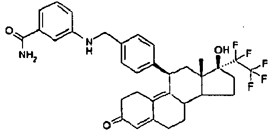 | 3-({4-[(11β,17β)-17-Hydroxy-3-oxo-17-(pentafluorethyl)estra-4,9-dien-11-yl]benzyl}amino)benzamid | 616, 1.29 min. |
| 54 | 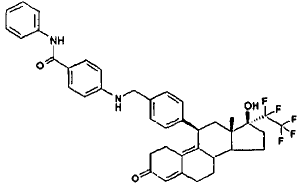 | 4-({4-[(11β,17β)-17-Hydroxy-3-oxo-17-(pentafluorethyl)estra-4,9-dien-11-yl]benzyl}amino)-N-phenylbenzamid | 692, 1.41 min. |

### Progesteronerezeptor-antagonistische Wirkung in stabilen Transfektanten humaner Neuroblastoma-Zellen (SK-N-MC Zellen) mit dem humanem Progesteron A- oder Progesteron B-Rezeptor und einem MTV-LUC Reporter Konstrukt

SK-N-MC Zellen (humane Neuroblastoma Zellen), die stabil mit Plasmiden transfiziert sind, welche den humanen Progesteronrezeptor-B (pRChPR-B-neo) oder den humanen Progesteronrezeptor-A (pRChPR-A-neo) und ein Reporterkonstrukt (pMMTV-LUC) exprimieren, wurden 24 Stunden entweder in Abwesenheit (Negativkontrolle) oder in Gegenwart von steigenden Mengen der jeweiligen Testverbindung (0.01 nmol/l, 0.1 nmol/l, 1 nmol/l, 10 nmol/l, 100 nmol/l und 1 µmol/l) inkubiert, um die agonistische Wirksamkeit zu bestimmen. Als Positivkontrolle der Reportergeninduktion wurden die Zellen mit dem synthetischen Gestagen Promegeston (0.01 nmol/l, 0.1 nmol/l, 1 nmol/l, 10 nmol/l, 100 nmol/l and 1 µmol/l) behandelt. Zur Bestimmung der antagonistischen Aktivität wurden die Zellen mit 0.1 nmol/l Promegeston und zusätzlich mit steigenden Mengen der jeweiligen Testverbindung (0.01 nmol/l, 0.1 nmol/l, 1 nmol/l, 10 nmol/l, 100 nmol/l and 1 µmol/l) behandelt. Die Aktivität des Reportergens LUC (LUC = Luciferase) wurde in den Zelllysaten bestimmt und als RLU (relative light units) gemessen. Alle Meßwerte werden angegeben als % Wirksamkeit und als EC₅₀ bzw. IC₅₀ Konzentrationen.
a) agonistische Aktivität:
   Keine der genannten Testverbindungen zeigt eine agonistische Aktivität.
b) antagonistische Aktivitat:
   Alle genannten Verbindungen zeigen eine 100%ige antagonistische Wirksamkeit.
      Die antagonistische Wirkstärke der Verbindungen ist in Tabelle 1 zusammengefasst.

**Tabelle 1: Antagonistische Wirkstärke der Verbindungen**

| Bsp. | PR-A IC₅₀ [nM] | PR-B IC₅₀ [nM] | Bsp. | PR-A IC₅₀ [nM] | PR-B IC₅₀ [nM] | Bsp. | PR-A IC₅₀ [nM] | PR-B IC₅₀ [nM] |
|---|---|---|---|---|---|---|---|---|
| 1 | 7.5 | 8.2 | 26 | 4.65 | n.b. | 51 | 64.7 | n.b. |
| 2 | 3.5 | 2.8 | 27 | 3.52 | n.b. | 52 | 6.50 | n.b. |
| 3 | 0.11 | 0.15 | 28 | 5.05 | n.b. | 53 | 0.84 | n.b. |
| 4 | 1.4 | 2.1 | 29 | 1.19 | n.b. | 54 | 1.28 | n.b. |
| 5 | 1.2 | 0.99 | 30 | 2.35 | n.b. | | | |
| 6 | 0.33 | 0.3 | 31 | 4.52 | n.b. | | | |
| 7 | 0.1 | 0.1 | 32 | 0.78 | n.b. | | | |
| 8 | 0.1 | 0.3 | 33 | 5.57 | n.b. | | | |
| 9 | 1.3 | 0.98 | 34 | 5.55 | n.b. | | | |
| 10 | 0.88 | 0.12 | 35 | 0.68 | n.b. | | | |
| 11 | 0.095 | 0.095 | 36 | 33.7 | n.b. | | | |
| 12 | 0.17 | 0.1 | 37 | 2.33 | n.b. | | | |
| 13 | 0.092 | 0.1 | 38 | 0.66 | n.b. | | | |
| 14 | 0.1 | 0.094 | 39 | 1.77 | n.b. | | | |
| 15 | 53 | 60 | 40 | 0.49 | n.b. | | | |
| 16 | 2.7 | 2.8 | 41 | 11.2 | n.b. | | | |
| 17 | 0.56 | 0.57 | 42 | 0.52 | n.b. | | | |
| 18 | 0.51 | n.b. | 43 | 2.88 | n.b. | | | |
| 19 | 0.94 | n.b. | 44 | 3.06 | n.b. | | | |
| 20 | 1.64 | n.b. | 45 | 1.65 | n.b. | | | |
| 21 | 6.06 | n.b. | 46 | 37.4 | n.b. | | | |
| 22 | 2.52 | n.b. | 47 | 2.71 | n.b. | | | |
| 23 | 2.07 | n.b. | 48 | 1.28 | n.b. | | | |
| 24 | 51 | n.b. | 49 | 1.29 | n.b. | | | |
| 25 | 3.06 | n.b. | 50 | 1.20 | n.b. | | | |

| | | | | | | | | |
|---|---|---|---|---|---|---|---|---|
| n.b.: nicht bestimmt | | | | | | | | |

### Abortivtest an weiblichen Ratten

Die antagonistische Wirkung der erfindungsgemäßen Verbindungen wurde an trächtigen Ratten (6 Ratten pro Gruppe) an Tag (d) 5 bis 7 post coitum (p.c.) unter herkömmlichen Haltungs- und Fütterungsbedingungen getestet.

Nach erfolgreicher Anpaarung, wurden die trächtigen Tiere (Vorhandensein von Spermien im Vaginalabstrich an Tag 1 der Schwangerschaft = d1 p.c.) randomisiert und auf die Behandlungs- und Kontrollgruppe aufgeteilt. Die Tiere erhielten dann subkutan oder oral je 0,15; 0,5; 1,5 oder 5 mg/kg der Testverbindung oder 1,0 ml/kg Vehikel (Benzylbenzoat/Rhizinusöl: 1+4 [v/v]) täglich von Tag 5 bis Tag 7 (d5 - d7 p.c.).

Die Autopsie wurde an Tag 9 (d9 p.c.) durchgeführt. Als Kenngröße der Progesteronrezeptor-antagonistischen Wirkung wurde der Uterus auf das Vorhandensein von Nidationsstellen untersucht. Dabei wurde das völlige Fehlen, aber auch das Vorhandensein pathologischer, hämorrhagischer oder sonst abnormer Nidationsstellen an Tag 9 (d9 p.c.) als Abort gewertet. Die Ergebnisse der Tests sind in Tabelle 3 dargestellt. Die Testverbindung zeigt in allen Dosierung einen vollen Effekt.

**Tab. 2: Ergebnisse an der Ratte (Terminierung der frühen Schwangerschaft)**

| Testverbindung nach | Tagesdosis [mg/kg] p.o. | Abortrate [%] |
|---|---|---|
| Vehikel | | 0 |
| Beispiel 11 | 0,5 | 100 |
| (11β,17β)-11-{4-[(4-Acetyl-piperazin-1-yl)methyl]-phenyl}-17-hydroxy-17-(pentafluorethyl)estra-4,9-dien-3-on | 1,5 | 100 |
| | 5,0 | 100 |
| Beispiel 12 | 0,5 | 100 |
| (11β,17β)-17-Hydroxy-11-(4-{[4-(methylsulfonyl)piperazin-1-yl]methyl}phenyl)-17-(pentafluorethyl)estra-4,9-dien-3-on | 1,5 | 100 |
| | 5,0 | 100 |

| | | |
|---|---|---|
| p.o.: per oral | | |

## Patentansprüche

1. 17-Hydroxy-17-pentafluorethyl-estra-4,9(10)-dien-11-aryl-Derivate der allgemeinen Formel I 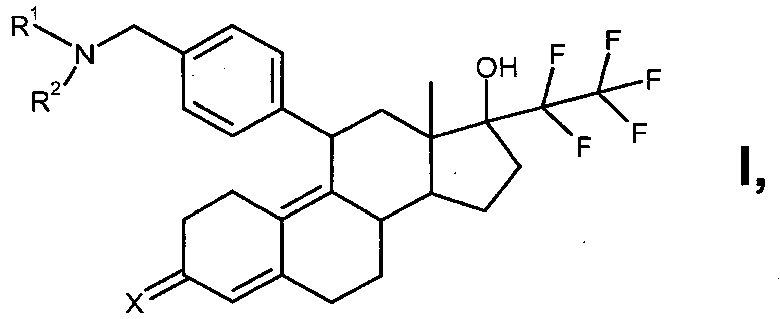 worin
R¹ und R² gleich oder verschieden sind und für
Wasserstoff,
einen gegebenenfalls Dimethylamin-substituierten C₁-C₁₀-Alkylrest, einen gegebenenfalls ein-, zwei- oder mehrfach mit Halogen (-F, -Cl, -Br, -I),-OH, -O-Alkyl, -C(O)OH, -C(O)OAlkyl, -C(O)NH₂, -C(O)NHAlkyl, -C(O)NDialkyl, -C(O)NHAryl, -C(O)NHHeteroaryl, -NH₂, -NH(C₁-C₁₀-Alkyl), -N(C₁-C₁₀-Alkyl)₂, insbesondere -N(CH₃)₂, -NHC(O)Alkyl, -NO₂, -N₃, -CN, -C₁-C₁₀-Alkyl, -C₁-C₁₀-Perfluor-Alkyl, -C₁-C₁₀-Acyl, -C₁-C₁₀-Acyloxy, -SO₂NH₂, -SO₂NHAlkyl oder-SO₂NDialkyl substituierten 6-10-gliedrigen Arylrest,
einen gegebenenfalls ein-, zwei- oder mehrfach mit den oben genannten Substituenten des 6-10-gliedrigen Arylrests substituierten 5-10-gliedrigen Heteroarylrest,
einen am Arylring gegebenenfalls ein-, zwei- oder mehrfach mit den oben genannten Substituenten des 6-10-gliedrigen Arylrests substituierten C₁-C₆-Arylalkylrest oder
einen am Heteroarylring gegebenenfalls ein-, zwei- oder mehrfach mit den oben genannten Substituenten des 6-10-gliedrigen Arylrests substituierten C₁-C₆-Heteroarylalkylrest stehen
oder aber
R¹ und R² gemeinsam Bestandteil eines gegebenenfalls am Kohlenstoff Alkyl-, Carboxyl-, Alkoxycärbonyl-, Alkylcarbonyl-, Aminocarbonyl-, Arylalkyl-, Heteroarylalkyl-, Aminoalkyl-substituierten bzw. am Stickstoff Alkyl-, Alkanoyl-, Carboxyl-, Alkoxycarbonyl-, Phenyl-, Phenylalkyl-, Pyridinyl-, Pyrimidinyl-, Pyrazinyl-, Sulfonyl-, Benzoyl-, Alkylsulfonyl-, Arylsulfonyl-, Aminocarbonyl-, Aminocarbonylalkyl-, Arylalkyl-, Heteroarylalkyl-, Heterocyclylalkyl- und Aminoalkyl-substituierten 3-10-gliedrigen Ringes sind, der gegebenenfalls Stickstoff-, Sauerstoff- oder Schwefelatome, die gegebenenfalls zum Sulfoxid oder Sulfon oxidiert sein können enthält, wobei an den 3-10-gliedrigen Ring gegebenenfalls ein Aromat ankondensiert sein kann,
X für ein Sauerstoffatom oder, NOR³ oder NNHSO₂R³,
R³ ausgewählt ist aus der Gruppe, umfassend Wasserstoff, C₁-C₁₀-Alkyl, Aryl
und ihre Salze, Solvate oder Solvate der Salze, einschließlich aller Kristallmodifikationen.

2. Verbindung gemäß Anspruch 1 der allgemeinen Formel II 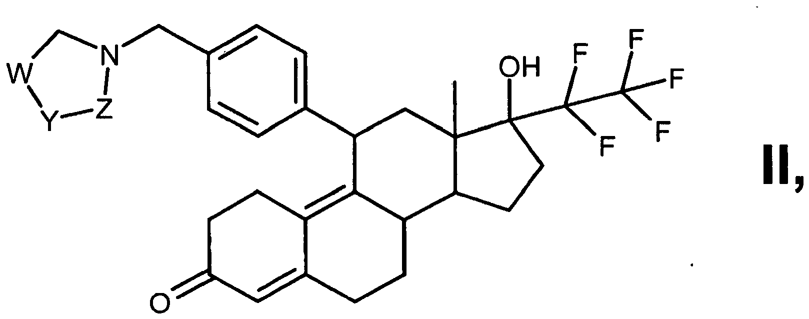 worin
Z -CH₂-, -CH₂-CH₂- oder -CH₂-CH₂-CH₂-,
W -CH₂-,
Y -CHR⁴-, -NR⁵-, -O- oder -SO₂- oder
W und Y gemeinsam Bestandteil eines ankondensierten aromatischen Ringes sind,
R⁴ und R⁵ Wasserstoff, -C₁-C₄-Alkyl, -(CH₂)ₘ-Phenyl, -(CH₂)ₘ-Pyridinyl, -(CH₂)ₘ-Pyrazinyl, -(CH₂)ₘ-NR⁷R⁸, -(CH₂)ₘ-C(O)-R⁶ mit m = 0, 1, 2 oder 3 oder - SO₂-C₁-C₄-Alkyl,
R⁶ -OH, -C₁-C₄-Alkyl, -O-C₁-C₄-Alkyl, -Phenyl oder -NR⁷R⁸ und
R⁷, R⁸ unabhängig voneinander Wasserstoff oder -C₁-C₄-Alkyl bedeuten oder gemeinsam Bestandteil eines 5- bis 7-gliedrigen Ringes sind
und ihre Salze, Solvate oder Solvate der Salze, einschließlich aller Kristallmodifikationen.

3. Verbindung gemäß Anspruch 2
worin
Z -CH₂-CH₂- oder -CH₂-CH₂-CH₂-,
W -CH₂-,
Y -CHR⁴-, -NR⁵-,
R⁴ und R⁵ -C(O)-R⁶ oder -SO₂-C₁-C₄-Alkyl und
R⁶ -OH, -C₁-C₄-Alkyl, -O-C₁-C₄-Alkyl bedeutet
und ihre Salze, Solvate oder Solvate der Salze, einschließlich aller Kristallmodifikationen.

4. Verbindung gemäß Anspruch 1
worin
R¹ und R² gleich oder verschieden sind und unabhängig voneinander C₁-C₄-Alkyl-, Di-C₁-C₄-Alkylamino-C₁-C₄-Alkyl-, einen gegebenenfalls Halogen-, C₁-C₄-Alkoxy-, - SO₂NH₂, -C(O)NH₂, -C(O)O-C₁-C₄-Alkyl, -NH-C(O)-C₁-C₄-Alkyl oder -C(O)-NH-Phenyl-substituierten Phenyl- oder C₁-C₄-Alkylphenylrest oder einen gegebenenfalls Halogen-, C₁-C₄-Alkoxy-, -SO₂NH₂, -C(O)NH₂, -C(O)O-C₁-C₄-Alkyl, -NH-C(O)-C₁-C₄-Alkyl oder -C(O)-NH-Phenyl-substituierten Pyridyl- oder C₁-C₄-Alkylpyridylrest bedeuten,
und ihre Salze, Solvate oder Solvate der Salze, einschließlich aller Kristallmod ifikationen.

5. Verbindung gemäß Anspruch 4 worin
R¹ Wasserstoff oder C₁-C₄-Alkyl und
R² Wasserstoff;
C₁-C₄-Alkyl,
Dimethylamino-C₁-C₄-Alkyl,
einen gegebenenfalls Halogen-, Alkoxy-, -SO₂NH₂, -C(O)NH₂, -C(O)O-C₁-C₄-Alkyl, -NH-C(O)-C₁-C₄-Alkyl oder -C(O)-NH-Phenyl-substituierten Phenylrest, -C₁-C₄-Alkylphenyl,
Pyridyl- oder
-C₁-C₄-Alkylpyridyl bedeutet
und ihre Salze, Solvate oder Solvate der Salze, einschließlich aller Kristallmodifikationen.

6. Verbindung gemäß Anspruch 4 worin R¹ Wasserstoff, Methyl oder Ethyl bedeutet, sowie deren Salze, Solvate oder Solvate der Salze, einschließlich aller Kristallmodifikationen.

7. Verbindungen gemäß Anspruch 4 worin R²-CH₃, -CH₂CH₃, -CH₂-CH₂-N(CH₃)₂, einen gegebenenfalls am Phenylring Cl-, -OCH₃, -SO₂NH₂, -C(O)NH₂, -CO₂CH₃,-NH-C(O)CH₃ oder -C(O)-NH-Phenyl-substituierten -(CH₂)ₙ-Phenylrest oder einen-(CH₂)ₙ-Pyridyl mit n = 0, 1, 2 bedeutet sowie deren Salze, Solvate oder Solvate der Salze, einschließlich aller Kristallmodifikationen.

8. Verbindungen gemäß einem der Ansprüche 4, 6 oder 7 worin
R¹ Wasserstoff, Methyl oder Ethyl und
R² -CH₃, -CH₂CH₃, -CH₂-CH₂-N(CH₃)₂, einen gegebenenfalls am Phenylring Cl-, -OCH₃, -SO₂NH₂, -C(O)NH₂, -C(O)OCH₃, -NH-C(O)CH₃ oder -C(O)-NH-Phenyl-substituierten -(CH₂)ₙ-Phenylrest oder einen -(CH₂)ₙ-Pyridyl mit n = 0, 1, 2 bedeutet
und deren Salze, Solvate oder Solvate der Salze, einschließlich aller Kristallmodifikationen.

9. Die Verbindungen nach einem der vorstehenden Ansprüche, nämlich
(11β,17β)-11-{4-[(Dimethylamino)-methyl]phenyl}-17-hydroxy-17-(pentafluorethyl)estra-4,9-dien-3-on
(11β, 17β)-17-Hydroxy-11-{4-[(4-methylpiperazin-1-yl)methyl]phenyl}-17-(pentafluorethyl)estra-4,9-dien-3-on
tert-Butyl-4-{4-[(11β,17β)-17-hydroxy-3-oxo-17-(pentafluorethyl)estra-4,9-dien-11-yl]benzyl}piperazin-1-carboxylat
(11β, 17β)-17-Hydroxy-17-(pentafluorethyl)-11-[4-(piperidin-1-ylmethyl)phenyl]estra-4,9-dien-3-on
Methyl-1-{4-[(11β, 17β)-17-hydroxy-3-oxo-17-(pentafluorethyl)estra-4,9-dien-11-yl]benzyl}piperidin-4-carboxylat
tert-Butyl-4-{4-[(11β,17β)-17-hydroxy-3-oxo-17-(pentafluorethyl)estra-4,9-dien-11-yl]benzyl}-1,4-diazepan-1-carboxylat
(11β,17β)-17-Hydroxy-11-[4-(morpholin-4-ylmethyl)phenyl]-17-(pentafluorethyl)estra-4,9-dien-3-on
(11β,17β)-11-{4-[(1,1-Dioxidothiomorpholin-4-yl)methyl]phenyl}-17-hydroxy-17-(pentafluorethyl)estra-4,9-dien-3-on
(11β,17β)-17-Hydroxy-17-(pentafluorethyl)-11-(4-{[(2-phenylethyl)amino]methyl}phenyl)estra-4,9-dien-3-on
(11β,17β)-17-Hydroxy-17-(pentafluorethyl)-11-[4-(piperazin-1-ylmethyl)phenyl]estra-4,9-dien-3-on
(11β,17β)-11-{4-[(4-Acetylpiperazin-1-yl)methyl]phenyl}-17-hydroxy-17-(pentafluorethyl)estra-4,9-dien-3-on
(11β,17β)-17-Hydroxy-11-(4-{[4-(methylsulfonyl)piperazin-1-yl]methyl}phenyl)-17-(pentafluorethyl)estra-4,9-dien-3-on
(11β,17β)-11-{4-[(4-Benzoylpiperazin-1-yl)methyl]phenyl}-17-hydroxy-17-(pentafluorethyl)estra-4,9-dien-3-on
(11β,17β)-11-(4-{[4-(2,2-Dimethylpropanoyl)piperazin-1-yl]methyl}phenyl)-17-hydroxy-17-(pentafluorethyl)estra-4,9-dien-3-on
1-{4-[(11β,17β)-17-Hydroxy-3-oxo-17-(pentafluorethyl)estra-4,9-dien-11-yl]benzyl}piperidin-4-carbonsäure
(11β,17β)-11-[4-(1,4-Diazepan-1-ylmethyl)phenyl]-17-hydroxy-17-(pentafluorethyl)estra-4,9-dien-3-on
(11β,17β)-11-{4-[(4-Acetyl-1,4-diazepan-1-yl)methyl]phenyl}-17-hydroxy-17-(pentafluorethyl)estra-4,9-dien-3-on
4-({4-[(11β,17β)-17-Hydroxy-3-oxo-17-(pentafluorethyl)estra-4,9-dien-11-yl]benzyl}amino)benzolsulfonamid
(11β,17β)-17-Hydroxy-17-(pentafluorethyl)-11-{4-[(pyridin-3-ylamino)methyl]phenyl}estra-4,9-dien-3-on
(11β,17β)-11-[4-(Anilinomethyl)phenyl]-17-hydroxy-17-(pentafluorethyl)estra-4,9-dien-3-on
(11β,17β)-17-Hydroxy-11-(4-{[(2-methoxyphenyl)amino]methyl}phenyl)-17-(pentafluorethyl)estra-4,9-dien-3-on
(11β,17β)-17-Hydroxy-11-(4-{[(3-methoxyphenyl)amino]methyl}phenyl)-17-(pentafluorethyl)estra-4,9-dien-3-on
(11β,17β}-11-(4-{[(4-Chlorphenyl)amino]methyl}phenyl)-17-hydroxy-17-(pentafluorethyl)estra-4,9-dien-3-on
(11β,17β)-11-{4-[(Diethylamino)methyl]phenyl}-17-hydroxy-17-(pentafluorethyl)estra-4,9-dien-3-on
(11β,17β)-17-Hydroxy-17-(pentafluorethyl)-11-{4-[(4-phenylpiperazin-1-yl)methyl]phenyl}estra-4,9-dien-3-on
(11β,17β)-17-Hydroxy-11-(4-{[methyl(pyridin-4-ylmethyl)amino]methyl}phenyl)-17-(pentafluorethyl)estra-4,9-dien-3-on
(11β,17β)-11-[4-({[2-(Dimethylamino)ethyl](methyl)amino}methyl)phenyl]-17-hydroxy-17-(pentafluorethyl)estra-4,9-dien-3-on
Methyl-2-({4-[(11β,17β)-17-hydroxy-3-oxo-17-(pentafluorethyl)estra-4,9-dien-11-yl]benzyl}amino)benzoat
Methyl-4-({4-[(11β,17β)-17-hydroxy-3-oxo-17-(pentafluorethy)estra-4,9-dien-11-yl]benzyl}amino)benzoat
(11β,17β)-11-[4-(3,4-Dihydroisochinolin-2(1H)-ylmethyl)phenyl]-17-hydroxy-17-(pentafluorethyl)estra-4,9-dien-3-on
(11β,17β)-11-{4-[(4-Benzylpiperazin-1-yl)methyl]phenyl}-17-hydroxy-17-(pentafluorethyl)estra-4,9-dien-3-on
4-({4-[(11β,17β)-17-Hydroxy-3-oxo-17-(pentafluorethyl)estra-4,9-dien-11-yl]benzyl}amino)benzamid
(11β,17β)-17-Hydroxy-11-(4-{[methyl(2-phenylethyl)amino]methyl}phenyl)-17-(pentafluorethyl)estra-4,9-dien-3-on
(11β,17β)-17-Hydroxy-17-(pentafluorethyl)-11-(4-{[4-(2-phenylethyl)piperazin-1-yl]methyl}phenyl)estra-4,9-dien-3-on
(11β,17β)-17-Hydroxy-17-(pentafluorethyl)-11-(4-{[4-(pyrazin-2-yl)piperazin-1-yl]methyl}phenyl)estra-4,9-dien-3-on
(11β,17β)-17-Hydroxy-17-(pentafluorethyl)-11-[4-({4-[2-(pyrrolidin-1-yl)ethyl]piperazin-1-yl}methyl)phenyl]estra-4,9-dien-3-on
(11β,17β)-17-Hydroxy-17-(pentafluorethyl)-11-(4-{[4-(pyridin-4-ylmethyl)piperazin-1-yl]methyl}phenyl)estra-4,9-dien-3-on
N-[4-({4-[(11β,17β)-17-Hydroxy-3-oxo-17-(pentafluorethyl)estra-4,9-dien-11-yl]benzyl}amino)phenyl]acetamid
Methyl-3-({4-[(11β,17β)-17-hydroxy-3-oxo-17-(pentafluorethyl)estra-4,9-dien-11-yl]benzyl}amino)benzoat
3-({4-[(11β,17β)-17-Hydroxy-3-oxo-17-(pentafluorethyl)estra-4,9-dien-11-yl]benzyl}amino)benzolsulfonamid
(11β,17β)-17-Hydroxy-17-(pentafluorethyl)-11-{4-[(4-phenylpiperidin-1-yl)methyl]phenyl}estra-4,9-dien-3-on
4-{4-[(11β,17β)-17-Hydroxy-3-oxo-17-(pentafluorethyl)estra-4,9-dien-11-yl]benzyl}-N,N-dimethylpiperazin-1-carboxamid
(11β,17β)-17-Hydroxy-17-(pentafluorethyl)-11-(4-{[4-(pyridin-2-ylmethyl)piperazin-1-yl]methyl}phenyl)estra-4,9-dien-3-on
(11β,17β)-17-Hydroxy-17-(pentafluorethyl)-11-(4-{[4-(pyridin-3-ylmethyl)piperazin-1-yl]methyl}phenyl)estra-4,9-dien-3-on
2-(4-{4-[(11β,17β)-17-Hydroxy-3-oxo-17-(pentafluorethyl)estra-4,9-dien-11-yl]benzyl}piperazin-1-yl)-N-methylacetamid
(11β,17β)-17-Hydroxy-17-(pentafluorethyl)-11-[4-(pyrrolidin-1-ylmethyl)phenyl]estra-4,9-dien-3-on
(11β,17β)-17-Hydroxy-17-(pentafluorethyl)-11-(4-{[(pyridin-4-ylmethyl)amino]methyl}phenyl)estra-4,9-dien-3-on
(11β,17β)-17-Hydroxy-11-(4-{[methyl(phenyl)amino]methyl}phenyl)-17-(pentafluorethyl)estra-4,9-dien-3-on
2-({4-[(11β,17β)-17-Hydroxy-3-oxo-17-(pentafluorethyl)estra-4,9-dien-11-yl]benzyl}amino)benzamid
(11β,17β)-17-Hydroxy-17-(pentafluorethyl)-11-(4-{[4-(pyridin-4-yl)piperazin-1-yl]methyl}phenyl)estra-4,9-dien-3-on
(11β,17β)-11-[4-({4-[2-(Dimethylamino)ethyl]piperazin-1-yl}methyl)phenyl]-17-hydroxy-17-(pentafluorethyl)estra-4,9-dien-3-on
(11β,17β)-17-Hydroxy-11-[4-({methyl[2-(pyridin-2-yl)ethyl]amino}methyl)phenyl]-17-(pentafluorethyl)estra-4,9-dien-3-on
3-({4-[(11β,17β)-17-Hydroxy-3-oxo-17-(pentafluorethyl)estra-4,9-dien-11-yl]benzyl}amino)benzamid
4-({4-[(11β,17β)-17-Hydroxy-3-oxo-17-(pentafluorethyl)estra-4,9-dien-11-yl]benzyl}amino)-N-phenylbenzamid
und ihre Salze, Solvate oder Solvate der Salze, einschließlich aller Kristallmodifikationen.

10. Eine Verbindung gemäß einem der vorstehenden Ansprüche zur Behandlung und Prophylaxe von Krankheiten.

11. Eine Verbindung gemäß einem der Ansprüche 1 - 9 zur Behandlung und Prophylaxe von Uterusfibroiden, der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

12. Verwendung einer Verbindung nach einem der Ansprüche 1 - 9 zur Herstellung eines Arzneimittels zur Behandlung und/oder Prophylaxe von Krankheiten.

13. Verwendung einer Verbindung nach einem der Ansprüche 1 - 9 zur Herstellung eines Arzneimittels zur Behandlung und/oder Prophylaxe von Uterusfibroiden, der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

14. Arzneimittel enthaltend eine Verbindung, wie in einem der Ansprüche 1 - 9 definiert, in Kombination mit einem weiteren Wirkstoff.

15. Arzneimittel nach Anspruch 14 in Kombination mit einem inerten, nichttoxischen, pharmazeutisch geeigneten Hilfsstoff.

16. Arzneimittel nach Anspruch 15 zur Behandlung und/oder Prophylaxe von Uterusfibroiden, der Endometriose, schweren Menstruationsblutungen, Meningiomen, hormonabhängigen Mammakarzinomen und von mit der Menopause assoziierten Beschwerden oder zur Fertilitätskontrolle und Notfallkontrazeption.

## Claims

1. 17-Hydroxy-17-pentafluoroethyl-estra-4,9(10)-dien-11-aryl derivates of the general formula I 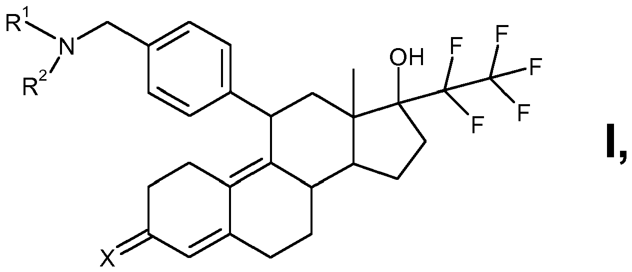 in which
R¹ and R² are identical or different and are hydrogen,
an optionally dimethylamine-substituted C₁-C₁₀-alkyl radical,
a 6-10-membered aryl radical optionally mono- , di- or polysubstituted with halogen (-F, -Cl, -Br, -I), -OH, -0-alkyl, -C(O)OH, -C(O)Oalkyl, -C(O)NH₂, -C(O)NHalkyl, -C(O)Ndialkyl, -C(O)NHaryl, -C(O)NHheteroaryl, -NH₂, -NH (C₁-C₁₀-alkyl), -N(C₁-C₁₀-alkyl)₂, in particular -N(CH₃)₂, -NHC(O)alkyl, -NO₂, -N₃, -CN, -C₁-C₁₀-alkyl, -C₁-C₁₀-perfluoroalkyl, -C₁-C₁₀-acyl, -C₁-C₁₀-acyloxy, -SO₂NH₂, -SO₂NHalkyl or -SO₂Ndialkyl,
a 5-10-membered heteroaryl radical optionally mono-, di- or polysubstituted with the aforementioned substituents of the 6-10-membered aryl radical,
a C₁-C₆-arylalkyl radical optionally mono-, di- or polysubstituted on the aryl ring with the aforementioned substituents of the 6-10-membered aryl radical or
a C₁-C₆-heteroarylalkyl radical optionally mono-, di- or polysubstituted on the heteroaryl ring with the aforementioned substituents of the 6-10-membered aryl radical,
or else
R¹ and R² are together a constituent of a 3-10-membered ring which is optionally substituted on the carbon by alkyl, carboxyl, alkoxycarbonyl, alkylcarbonyl, aminocarbonyl, arylalkyl, heteroarylalkyl, aminoalkyl and/or on the nitrogen by alkyl, alkanoyl, carboxyl, alkoxycarbonyl, phenyl, phenylalkyl, pyridinyl, pyrimidinyl, pyrazinyl, sulphonyl, benzoyl, alkylsulphonyl, arylsulphonyl, aminocarbonyl, aminocarbonylalkyl, arylalkyl, heteroarylalkyl, heterocyclylalkyl and aminoalkyl, and which optionally contains nitrogen, oxygen or sulphur atoms which can optionally be oxidized with the sulphoxide or sulphone, where an aromatic can optionally be fused onto the 3-10-membered ring,
X is an oxygen atom or NOR³ or NNHSO₂R³,
R³ is selected from the group comprising hydrogen, C₁-C₁₀-alkyl, aryl
and their salts, solvates or solvates of the salts, including all crystal modifications.

2. Compound according to Claim 1 of the general formula II 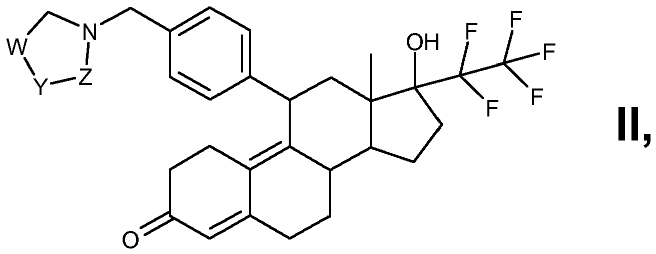 in which
Z is -CH₂-, -CH₂-CH₂- or -CH₂-CH₂-CH₂-,
W is -CH₂-,
Y is -CHR⁴-, -NR⁵-, -0- or -SO₂- or
W and Y together are a constituent of a fused aromatic ring,
R⁴ and R⁵ are hydrogen, -C₁-C₄-alkyl, -(CH₂)ₘ-phenyl, -(CH₂)ₘ-pyridinyl, -(CH₂)ₘ-pyrazinyl, -(CH₂)ₘ-NR⁷R⁸, - (CH₂)ₘ-C(O)-R⁶ where m = 0, 1, 2 or 3 or -SO₂-C₁-C₄-alkyl,
R⁶ is -OH, -C₁-C₄-alkyl, -O-C₁-C₄-alkyl, -phenyl or -NR⁷R⁸ and
R⁷, R⁸ independently of one another are hydrogen or -C₁-C₄-alkyl or together are a constituent of a 5- to 7-membered ring
and its salts, solvates or solvates of the salts, including all crystal modifications.

3. Compound according to Claim 2
in which
Z is -CH₂-CH₂- or -CH₂-CH₂-CH₂-,
W is -CH₂-,
Y is -CHR⁴-, -NR⁵-,
R⁴ and R⁵ are -C(O)-R⁶ or -SO₂-C₁-C₄-alkyl and
R⁶ is -OH, -C₁-C₄-alkyl, -O-C₁-C₄-alkyl
and its salts, solvates or solvates of the salts, includnig all crystal modifications.

4. Compound according to Claim 1
in which
R¹ and R² are identical or different and, independently of one another, are C₁-C₄-alkyl radical, di-C₁-C₄-alkylamino-C₁-C₄-alkyl radical, an optionally halogen-, C₁-C₄-alkoxy-, -SO₂NH₂-, -C(O)NH₂-, -C(O)O-C₁-C₄-alkyl-, -NH-C(O)-C₁-C₄-alkyl- or -C(0)-NH-phenyl-substituted phenyl or C₁-C₄-alkylphenyl radical or an optionally halogen-, C₁-C₄-alkoxy-, -SO₂NH₂-, -C(O)NH₂-, -C (0) O-C₁-C₄-alkyl-, -NH-C (O)-C₁-C₄-alkyl- or -C(O)-NH-phenyl-substituted pyridyl or C₁-C₄-alkylpyridyl radical,
and its salts, solvates or solvates of the salts, including all crystal modifications.

5. Compound according to Claim 4 in which
R¹ is hydrogen or C₁-C₄-alkyl and
R² is hydrogen;
C₁-C₄-alkyl,
dimethylamino-C₁-C₄-alkyl,
an optionally halogen-, alkoxy-, -SO₂NH₂-, -C(O)NH₂-, -C(O)O-C₁-C₄-alkyl-, -NH-C(O)-C₁-C₄-alkyl- or -C(0)-NH-phenyl-substituted phenyl radical,
-C₁-C₄-alkylphenyl,
pyridyl or
-C₁-C₄-alkylpyridyl
and its salts, solvates or solvates of the salts, including all crystal modifications.

6. Compound according to Claim 4 in which R¹ is hydrogen, methyl or ethyl, and its salts, solvates or solvates of the salts, including all crystal modifications.

7. Compounds according to Claim 4 in which R² is -CH₃, -CH₂CH₃, -CH₂-CH₂-N (CH₃)₂, a -(CH₂)ₙ-phenyl radical optionally substituted on the phenyl ring with Cl-, -OCH₃, -SO₂NH₂, -C(O)NH₂, -CO₂CH₃, -NH-C(O)CH₃ or -C(O)-NH-phenyl, or a -(CH₂)ₙ-pyridyl where n = 0, 1, 2, and their salts, solvates or solvates of the salts, including all crystal modifications.

8. Compounds according to one of Claims 4, 6 or 7, in which
R¹ is hydrogen, methyl or ethyl and
R² is -CH₃, -CH₂CH₃, -CH₂-CH₂-N(CH₃)₂, a -(CH₂)ₙ-phenyl radical optionally substituted on the phenyl ring with Cl-, -OCH₃, -SO₂NH₂, -C(O)NH₂, -C(O)OCH₃, -NH-C(O)CH₃ or -C(O)-NH-phenyl, or a -(CH₂)ₙ-pyridyl where n = 0, 1, 2
and their salts, solvates or solvates of the salts, including all crystal modifications.

9. Compounds according to one of the preceding claims, namely
(11β,17β)-11-{4-[(dimethylamino)methyl]phenyl}-17-hydroxy-17-(pentafluoroethyl)estra-4,9-dien-3-one
(11β,17β)-17-hydroxy-11-{4-[(4-methylpiperazin-1-yl)methyl]phenyl}-17-(pentafluoroethyl)estra-4,9-dien-3-one
tert-butyl 4-{4-[(11β,17β)-17-hydroxy-3-oxo-17-(pentafluoroethyl)estra-4,9-dien-11-yl]benzyl}piperazine-l-carboxylate
(11β,17β)-17-hydroxy-17-(pentafluoroethyl)-11-[4-(piperidin-1-ylmethyl)phenyl]estra-4,9-dien-3-one methyl 1-{4-[(11β,17β)-17-hydroxy-3-oxo-17-(pentafluoroethyl)estra-4,9-dien-11-yl]benzyl}-piperidine-4-carboxylate
tert-butyl 4-{4-[(11β,17β)-17-hydroxy-3-oxo-17-(pentafluoroethyl)estra-4,9-dien-11-yl]benzyl}-1,4-diazepane-1-carboxylate
(11β,17β)-17-hydroxy-11-[4-(morpholin-4-ylmethyl)-phenyl]-17-(pentafluoroethyl)estra-4,9-dien-3-one
(11β,17β)-11-{4-[(1,1-dioxidothiomorpholin-4-yl)-methyl]phenyl}-17-hydroxy-17-(pentafluoroethyl)-estra-4,9-dien-3-one
(11β,17β)-17-hydroxy-17-(pentafluoroethyl)-11-(4-{[(2-phenylethyl)amino]methyl}phenyl)estra-4,9-dien-3-one
(11β,17β)-17-hydroxy-17-(pentafluoroethyl)-11-[4-(piperazin-1-ylmethyl)phenyl]estra-4,9-dien-3-one
(11β,17β)-11-{4-[(4-acetylpiperazin-l-yl)methyl]-phenyl}-17-hydroxy-17-(pentafluoroethyl)estra-4,9-dien-3-one
(11β,17β)-17-hydroxy-11-(4-{[4-(methylsulphonyl)-piperazin-1-yl]methyl}phenyl)-17-(pentafluoroethyl)estra-4,9-dien-3-one
(11β,17β)-11-{4-[(4-benzoylpiperazin-l-yl)methyl]-phenyl}-17-hydroxy-17-(pentafluoroethyl)estra-4,9-dien-3-one
(11β,17β)-11-(4-{[4-(2,2-dimethylpropanoyl)-piperazin-1-yl]methyl}phenyl)-17-hydroxy-17-(pentafluoroethyl)estra-4,9-dien-3-one
1-{4-[(11β,17β)-17-hydroxy-3-oxo-17-(pentafluoroethyl)estra-4,9-dien-11-yl]benzyl}piperidine-4-carboxylic acid
(11β,17β)-11-[4-(1,4-diazepan-1-ylmethyl)phenyl]-17-hydroxy-17-(pentafluoroethyl)estra-4,9-dien-3-one
(11β,17β)-11-{4-[(4-acetyl-1,4-diazepan-1-yl)-methyl]phenyl}-17-hydroxy-17-(pentafluoroethyl)-estra-4,9-dien-3-one
4-({4-[(11β,17β)-17-hydroxy-3-oxo-17-(pentafluoroethyl)estra-4,9-dien-11-yl]benzyl}amino)benzenesulphonamide
(11β,17β)-17-hydroxy-17-(pentafluoroethyl)-11-{4-[(pyridin-3-ylamino)methyl]phenyl}estra-4,9-dien-3-one
(11β,17β)-11-[4-(anilinomethyl)phenyl]-17-hydroxy-17-(pentafluoroethyl)estra-4,9-dien-3-one
(11β,17β)-17-hydroxy-11-(4-{[(2-methoxyphenyl)-amino]methyl}phenyl)-17-(pentafluoroethyl)estra-4,9-dien-3-one
(11β,17β)-17-hydroxy-11-(4-{[(3-methoxyphenyl)-amino]methyl}phenyl)-17-(pentafluoroethyl)estra-4,9-dien-3-one
(11β,17β)-11-(4-{[(4-chlorophenyl)amino]methyl}-phenyl)-17-hydroxy-17-(pentafluoroethyl)estra-4,9-dien-3-one
(11β,17β)-11-{4-[(diethylamino)methyl]phenyl}-17-hydroxy-17-(pentafluoroethyl)estra-4,9-dien-3-one
(11β,17β)-17-hydroxy-17-(pentafluoroethyl)-11-{4-[(4-phenylpiperazin-1-yl)methyl]phenyl}estra-4,9-dien-3-one
(11β,17β)-17-hydroxy-11-(4-{[methyl(pyridin-4-yl-methyl)amino]methyl}phenyl)-17-(pentafluoroethyl)-estra-4,9-dien-3-one
(11β,17β)-11-[4-({[2-(dimethylamino)ethyl](methyl)-amino}methyl)phenyl]-17-hydroxy-17-(pentafluoroethyl)estra-4,9-dien-3-one
methyl 2-({4-[(11β,17β)-17-hydroxy-3-oxo-17-(pentafluoroethyl)estra-4,9-dien-11-yl]benzyl}amino)-benzoate
methyl 4-({4-[(11β,17β)-17-hydroxy-3-oxo-17-(pentafluoroethyl)estra-4,9-dien-11-yl]benzyl}amino)-benzoate
(11β,17β)-11-[4-(3,4-dihydroisoquinolin-2(1H)-yl-methyl)phenyl]-17-hydroxy-17-(pentafluoroethyl)-estra-4,9-dien-3-one
(11β,17β)-11-{4-[(4-benzylpiperazin-l-yl)methyl]-phenyl}-17-hydroxy-17-(pentafluoroethyl)estra-4,9-dien-3-one
4-({4-[(11β,17β)-17-hydroxy-3-oxo-17-(pentafluoroethyl)estra-4,9-dien-11-yl]benzyl}amino)benzamide
(11β,17β)-17-hydroxy-11-(4-{[methyl(2-phenylethyl)-amino]methyl}phenyl)-17-(pentafluoroethyl)estra-4,9-dien-3-one
(11β,17β)-17-hydroxy-17-(pentafluoroethyl)-11-(4-{[4-(2-phenylethyl)piperazin-1-yl]methyl}phenyl)-estra-4,9-dien-3-one
(11β,17β)-17-hydroxy-17-(pentafluoroethyl)-11-(4-{[4-(pyrazin-2-yl)piperazin-1-yl]methyl}phenyl)-estra-4,9-dien-3-one
(11β,17β)-17-hydroxy-17-(pentafluoroethyl)-11-[4-({4-[2-(pyrrolidin-1-yl)ethyl]piperazin-1-yl}-methyl)phenyl]estra-4,9-dien-3-one
(11β,7β)-17-hydroxy-17-(pentafluoroethyl)-11-(4-{[4-(pyridin-4-ylmethyl)piperazin-1-yl]methyl}-phenyl)estra-4,9-dien-3-one
N-[4-({4-[(11β,17β)-17-hydroxy-3-oxo-17-(pentafluoroethyl)estra-4,9-dien-11-yl]benzyl}amino)-phenyl]acetamide
methyl 3-({4-[(11β,17β)-17-hydroxy-3-oxo-17-(pentafluoroethyl)estra-4,9-dien-11-yl]benzyl}amino)-benzoate
3-({4-[(11β,17β)-17-hydroxy-3-oxo-17-(pentafluoroethyl)estra-4,9-dien-11-yl]benzyl}amino)benzenesulphonamide
(11β,17β)-17-hydroxy-17-(pentafluoroethyl)-11-{4-[(4-phenylpiperidin-1-yl)methyl]phenyl}estra-4,9-dien-3-one
4-{4-[(11β,17β)-17-hydroxy-3-oxo-17-(pentafluoroethyl)estra-4,9-dien-11-yl]benzyl}-N,N-dimethylpiperazine-1-carboxamide
(11β,17β)-17-hydroxy-17-(pentafluoroethyl)-11-(4-{[4-(pyridin-2-ylmethyl)piperazin-1-yl]methyl}-phenyl)estra-4,9-dien-3-one
(11β,17β)-17-hydroxy-17-(pentafluoroethyl)-11-(4-{[4-(pyridin-3-ylmethyl)piperazin-1-yl]methyl}-phenyl)estra-4,9-dien-3-one
2-(4-{4-[(11β,17β)-17-hydroxy-3-oxo-17-(pentafluoroethyl)estra-4,9-dien-11-yl]benzyl}piperazin-1-yl)-N-methylacetamide
(11β,17β)-17-hydroxy-17-(pentafluoroethyl)-11-[4-(pyrrolidin-1-ylmethyl)phenyl]estra-4,9-dien-3-one
(11β,17β)-17-hydroxy-17-(pentafluoroethyl)-11-(4-{[(pyridin-4-ylmethyl)amino]methyl}phenyl)estra-4,9-dien-3-one
(11β,17β)-17-hydroxy-11-(4-{[methyl(phenyl)amino]-methyl}phenyl)-17-(pentafluoroethyl)estra-4,9-dien-3-one
2-({4-[(11β,17β)-17-hydroxy-3-oxo-17-(pentafluoroethyl)estra-4,9-dien-11-yl]benzyl}amino)benzamide
(11β,17β)-17-hydroxy-17-(pentafluoroethyl)-11-(4-{[4-(pyridin-4-yl)piperazin-1-yl]methyl}phenyl)-estra-4,9-dien-3-one
(11β,17β)-11-[4-({4-[2-(dimethylamino)ethyl]-piperazin-1-yl}methyl)phenyl]-17-hydroxy-17-(pentafluoroethyl)estra-4,9-dien-3-one
(11β,17β)-17-hydroxy-11-[4-({methyl[2-(pyridin-2-yl)ethyl]amino}methyl)phenyl]-17-(pentafluoroethyl)estra-4,9-dien-3-one
3-({4-[(11β,17β)-17-hydroxy-3-oxo-17-(pentafluoroethyl)estra-4,9-dien-11-yl]benzyl}amino)benzamide
4-({4-[(11β,17β)-17-hydroxy-3-oxo-17-(pentafluoroethyl)estra-4,9-dien-11-yl]benzyl}amino)-N-phenylbenzamide
and their salts, solvates and solvates of the salts, including all crystal modifications.

10. Compound according to one of the preceding claims for the treatment and prophylaxis of diseases.

11. Compound according to one of Claims 1-9 for the treatment and prophylaxis of uterus fibroids, endometriosis, heavy menstrual bleeding, meningiomas, hormone-dependent breast cancers and troubles associated with the menopause or for monitoring fertility and emergency contraception.

12. Use of a compound according to one of Claims 1-9 for producing a medicament for the treatment and/or prophylaxis of diseases.

13. Use of a compound according to one of Claims 1-9 for producing a medicament for the treatment and/or prophylaxis of uterus fibroids, endometriosis, heavy menstrual bleeding, mengiomas, hormone-dependent breast cancers and troubles associated with the menopause or for monitoring fertility and emergency contraception.

14. Medicament comprising a compound as defined in one of Claims 1-9 in combination with a further active ingredient.

15. Medicament according to Claim 14 in combination with an inert, nontoxic, pharmaceutically suitable auxiliary.

16. Medicament according to Claim 15 for the treatment and/or prophylaxis of uterus fibroids, endometriosis, heavy menstrual bleeding, meningiomas, hormone-dependent breast cancers and troubles associated with the menopause or for monitoring fertility and emergency contraception.

## Revendications

1. Dérivés 17-hydroxy-17-pentafluoroéthyl-estra-4,9(10)-dién-11-aryle de formule générale I 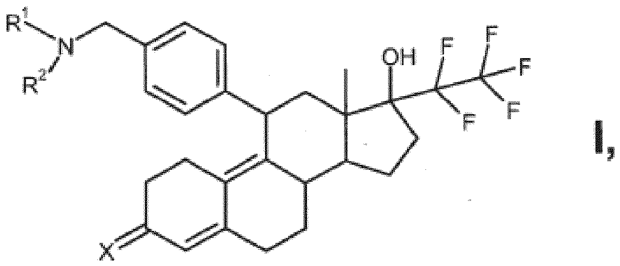 dans laquelle
R¹ et R² sont identiques ou différents et représentent un atome d'hydrogène,
un radical alkyle en C₁-C₁₀ éventuellement substitué par diméthylamino,
un radical aryle à 6-10 chaînons, éventuellement une, deux ou plus de deux fois substitué par halogéno (-F, -Cl, -Br, -I), -OH, -O-alkyle, -C(O)OH, -C(O)Oalkyle, -C(O)NH₂, -C(O)NHalkyle, -C(0)Ndialkyle, -C(0)NHaryle, -C(0)NHhétéroaryle, -NH₂, -NH (alkyle (C₁-C₁₀)), -N (alkyle (C₁-C₁₀))₂, en particulier -N(CH₃)₂, -NHC(O)alkyle, -NO₂, -N₃, -CN, alkyle en C₁-C₁₀, -perfluoro-alkyle(C₁-C₁₀), -acyle en C₁-C₁₀, -acyloxy (C₁-C₁₀), -SO₂NH₂, -SO₂NHalkyle ou -SO₂Ndialkyle,
un radical hétéroaryle à 5-10 chaînons éventuellement une, deux ou plus de deux fois substitué par les substituants indiqués ci-dessus du radical aryle à 6-10 chaînons,
un radical arylalkyle(C₁-C₆) éventuellement une, deux ou plus de deux fois substitué sur le cycle aryle par les substituants indiqués ci-dessus du radical aryle à 6-10 chaînons ou
un radical hétéroarylalkyle(C₁-C₆) éventuellement une, deux ou plus de deux fois substitué sur le cycle hétéroaryle par les substituants indiqués ci-dessus du radical aryle à 6-10 chaînons,
ou bien
R¹ et R² font partie ensemble d'un cycle à 3-10 chaînons, éventuellement substitué sur le carbone par alkyle, carboxy, alcoxycarbonyle, alkylcarbonyle, aminocarbonyle, arylalkyle, hétéroarylalkyle, aminoalkyle ou sur l'azote par alkyle, alcanoyle, carboxy, alcoxycarbonyle, phényle, phénylalkyle, pyridinyle, pyrimidinyle, pyrazinyle, sulfonyle, benzoyle, alkylsulfonyle, arylsulfonyle, aminocarbonyle, aminocarbonylalkyle, arylalkyle, hétéroarylalkyle, hétérocyclylalkyle et aminoalkyle, qui contient éventuellement des atomes d'azote, d'oxygène ou de soufre qui peuvent éventuellement être oxydés en le sulfoxyde ou la sulfone, un radical aromatique pouvant éventuellement être soudé au cycle à 3-10 chaînons,
X représente un atome d'oxygène ou NOR³ ou NNHSO₂R³,
R³ est choisi dans le groupe comprenant un atome d'hydrogène, un groupe alkyle en C₁-C₁₀, aryle
et leurs sels, produits de solvatation ou produits de solvatation des sels, y compris toutes les formes cristallines.

2. Composé selon la revendication 1 de formule générale II 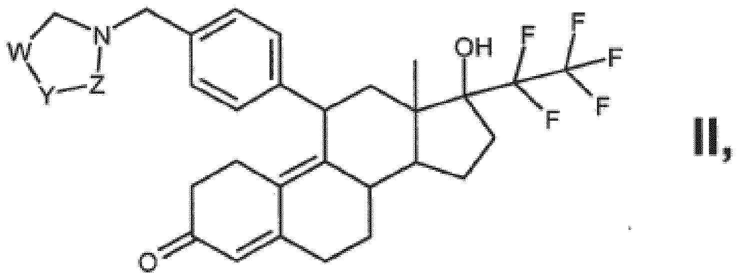 dans laquelle
Z représente -CH₂-, -CH₂-CH₂- ou -CH₂-CH₂-CH₂-,
W représente -CH₂-,
Y représente -CHR⁴-, -NR⁵-, -0- ou -SO₂- ou W et Y font ensemble partie d'un cycle aromatique condensé,
R⁴ et R⁵ représentent un atome d'hydrogène, un groupe alkyle en C₁-C₄, -(CH₂)ₘ-phényle, -(CH₂)ₘ-pyridinyle, -(CH₂)ₘ-pyrazinyle, -(CH₂)ₘ-NR⁷R⁸, -(CH₂)ₘ-C(O)-R⁶ où m = 0, 1, 2 ou 3 ou -SO₂-alkyle(C₁-C₄),
R⁶ représente -OH, alkyle en C₁-C₄, -O-alkyle(C₁-C₄), -phényle ou -NR⁷R⁸ et
R⁷, R⁸ représentent indépendamment l'un de l'autre un atome d'hydrogène ou un groupe alkyle en C₁-C₄ ou font partie ensemble d'un cycle à 5 à 7 chaînons
et ses sels, produits de solvatation ou produits de solvatation des sels, y compris toutes les formes cristallines.

3. Composé selon la revendication 2,
dans lequel
Z représente -CH₂-CH₂- ou -CH₂-CH₂-CH₂-,
W représente -CH₂-,
Y représente -CHR⁴-, -NR⁵-,
R⁴ et R⁵ représentent -C(O)-R⁶ ou -SO₂-alkyle(C₁-C₄) et
R6 représente -OH, alkyle en C₁-C₄, -O-alkyle(C₁-C₄), et ses sels, produits de solvatation ou produits de solvatation des sels, y compris toutes les formes cristallines.

4. Composé selon la revendication 1
dans lequel
R¹ et R² sont identiques ou différents et représentent indépendamment l'un de l'autre un groupe alkyle en C₁-C₄, dialkyl(C₁-C₄)amino-alkyle(C₁-C₄), un radical phényle ou alkyl(C₁-C₄)phényle éventuellement substitué par halogéno, alcoxy en C₁-C₄, -SO₂NH₂, -C(O)NH₂, -C(O)O-alkyle(C₁-C₄), -NH-C(O)-alkyle(C₁-C₄) ou -(CO)-NH-phényle ou un radical pyridyle ou alkyl(C₁-C₄)pyridyle éventuellement substitué par halogéno, alcoxy en C₁-C₄, -SO₂NH₂, -C(O)NH₂, -C(O)O-alkyle(C₁-C₄), -NH-C(O)-alkyle(C₁-C₄) ou -C(O)-NH-phényle,
et ses sels, produits de solvatation ou produits de solvatation des sels, y compris toutes les formes cristallines.

5. Composé selon la revendication 4
dans lequel
R¹ représente un atome d'hydrogène ou un groupe alkyle en C₁-C₄ et
R² représente un atome d'hydrogène ;
un groupe alkyle en C₁-C₄,
diméthylamino-alkyle(C₁-C₄),
un radical phényle éventuellement substitué par halogéno, alcoxy, -SO₂NH₂, -C(O)NH₂, -C(0)0-alkyle(C₁-C₄), -NH-C(O)-alkyle(C₁-C₄) ou -C(O)-NH-phényle
alkyl(C₁-C₄)phényle,
pyridyle ou
alkyl(C₁-C₄)pyridyle
et ses sels, produits de solvatation ou produits de solvatation des sels, y compris toutes les formes cristallines.

6. Composé selon la revendication 4, dans lequel R¹ représente un atome d'hydrogène, le groupe méthyle ou éthyle, ainsi que ses sels, produits de solvatation ou produits de solvatation des sels, y compris toutes les formes cristallines.

7. Composé selon la revendication 4, dans lequel R² représente -CH₃, -CH₂CH₃, -CH₂-CH₂-N(CH₃)₂, un radical -(CH₂)ₙ-phényle éventuellement substitué sur le cycle phényle par Cl-, -OCH₃, -SO₂NH₂, -C(O)NH₂, -CO₂CH₃, -NH-C(O)CH₃ ou -C(O)-NH-phényle ou un radical -(CH₂)ₙ-pyridyle où n = 0, 1, 2 ainsi que ses sels, produits de solvatation ou produits de solvatation des sels, y compris toutes les formes cristallines.

8. Composés selon l'une quelconque des revendications 4, 6 ou 7, dans lesquels
R¹ représente un atome d'hydrogène, le groupe méthyle ou éthyle et
R² représente -CH₃, -CH₂CH₃, -CH₂-CH₂-N(CH₃)₂, un radical -(CH₂)ₙ-phényle éventuellement substitué sur le cycle phényle par Cl-, -OCH₃, -SO₂NH₂, -C(O)NH₂, -C(O)OCH₃, -NH-C(O)CH₃ ou -C(O)-NH-phényle ou un radical -(CH₂)ₙ-pyridyle où n = 0, 1, 2
et leurs sels, produits de solvatation ou produits de solvatation des sels, y compris toutes les formes cristallines.

9. Les composés selon l'une quelconque des revendications précédentes, à savoir
(11β,17β)-11-{4-[(diméthylamino)-méthyl]phényl}-17-hydroxy-17-(pentafluoroéthyl)estra-4,9-dién-3-one
(11β,17β)-17-hydroxy-11-{4-[(4-méthylpipérazin-1-yl)méthyl]phényl}-17-(pentafluoroéthyl)estra-4,9-dién-3-one
4-{4-[(11β,17β)-17-hydroxy-3-oxo-17-(pentafluoroéthyl)-estra-4,9-dién-11-yl]benzyl}pipérazine-1-carboxylate de tert-butyle
(11β,17β)-17-hydroxy-17-(pentafluoroéthyl)-11-[4-(pipéridin-1-ylméthyl)phényl]estra-4,9-dién-3-one
1-{4-[(11β,17β)-17-hydroxy-3-oxo-17-(pentafluoroéthyl)-estra-4,9-dién-11-yl]benzyl}pipéridin-4-carboxylate de méthyle
4-{4[(11β,17β)-17-hydroxy-3-oxo-17-(pentafluoroéthyl)-estra-4,9-dién-11-yl]benzyl}-1,4-diazépane-1-carboxylate de tert-butyle
(11β,17β)-17-hydroxy-11-[4-(morpholin-4-ylméthyl)-phényl]-17-(pentafluoroéthyl)estra-4,9-dién-3-one
(11β,17β)-11-{4-[(1,1-dioxydothiomorpholin-4-yl)-méthyl]phényl}-17-hydroxy-17-(pentafluoroéthyl)estra-4,9-dién-3-one
(11β,17β)-17-hydroxy-17-(pentafluoroéthyl)-11-(4-{[(2-phényléthyl)amino]méthyl}phényl)estra-4,9-dién-3-one
(11β,17β)-17-hydroxy-17-(pentafluoroéthyl)-11-[4-(pipérazin-1-ylméthyl)phényl]estra-4,9-dién-3-one
(11β,17β)-11-{4-[(4-acétylpipérazin-1-yl)méthyl]phényl}-17-hydroxy-17-(pentafluoroéthyl)estra-4,9-dién-3-one
(11β,17β)-17-hydroxy-11-(4-{[4-(méthylsulfonyl)-pipérazin-1-yl]méthyl}phényl)-17-(pentafluoroéthyl)-estra-4,9-dién-3-one
(11β,17β)-11-{4-[(4-benzoylpipérazin-1-yl)méthyl]-phényl}-17-hydroxy-17-(pentafluoréthyl)estra-4,9-dién-3-one
(11β,17β)-11-(4-{[4-(2,2-diméthylpropanoyl)pipérazin-1-yl]méthyl}phényl)-17-hydroxy-17-(pentafluoroéthyl)-estra-4,9-dién-3-one
acide 1-{4-[(11β,17β)-17-hydroxy-3-oxo-17-(pentafluoroéthyl)estra-4,9-dién-11-yl]benzyl}pipéridin-4-carboxylique
(11β,17β)-11-[4-(1,4-diazépan-1-ylméthyl)phényl]-17-hydroxy-17-(pentafluoroéthyl)estra-4,9-dién-3-one
(11β,17β)-11-{4-[(4-acétyl-1,4-diazépan-1-yl)méthyl]-phényl}-17-hydroxy-17-(pentafluoroéthyl)estra-4,9-dién-3-one
4-({4-[(11β,17β)-17-hydroxy-3-oxo-17-(pentafluoroéthyl)estra-4,9-dién-11-yl]benzyl}amino)benzènesulfonamide
(11β,17β)-17-hydroxy-17-(pentafluoroéthyl)-11-{4-[(pyridin-3-ylamino)méthyl]phényl}estra-4,9-dién-3-one
(11β,17β)-11-[4-(anilinométhyl)phényl]-17-hydroxy-17-(pentafluoroéthyl)estra-4,9-dién-3-one
(11β,17β)-17-hydroxy-11-(4-{[(2-méthoxyphényl)amino]-méthyl}phényl)-17-(pentafluoroéthyl)estra-4,9-dién-3-one
(11β,17β)-17-hydroxy-11-(4-{[(3-méthoxyphényl)amino]-méthyl}phényl)-17-(pentafluoroéthyl)estra-4,9-dién-3-one
(11β,17β)-11-(4-{[(4-chlorophényl)amino]méthyl}phényl)-17-hydroxy-17-(pentafluoroéthyl)estra-4,9-dién-3-one
(11β,17β)-11-{4-[(diéthylamino)méthyl]phényl}-17-hydroxy-17-(pentafluoroéthyl)estra-4,9-dién-3-one
(11β,17β)-17-hydroxy-17-(pentafluoroéthyl)-11-{4-[(4-phénylpipérazin-l-yl)méthyl]phényl}estra-4,9-dién-3-one
(11β,17β)-17-hydroxy-11-(4-{[méthyl(pyridin-4-yl-méthyl)amino]méthyl}phényl)-17-(pentafluoroéthyl)estra-4,9-dién-3-one
(11β,17β)-11-[4-({[2-(diméthylamino)éthyl](méthyl)-amino}méthyl)phényl]-17-hydroxy-17-pentafluoroéthyl)-estra-4,9-dién-3-one
2-({4-[(11β,17β)-17-hydroxy-3-oxo-17-(pentafluoroéthyl)estra-4,9-dién-11-yl]benzyl}amino)benzoate de méthyle
4-({4-[(11β,17β)-17-hydroxy-3-oxo-17-(pentafluoroéthyl)estra-4,9-dién-11-yl]benzyl}amino)benzoate de méthyle
(11β,17β)-11-[4-(3,4-dihydroisoquinolin-2(1H)-ylméthyl)phényl]-17-hydroxy-17-(pentafluoroéthyl)estra-4,9-dién-3-one
(11β,17β)-11-{4-[(4-benzylpipérazin-1-yl)méthyl]-phényl}-17-hydroxy-17-(pentafluoroéthyl)estra-4,9-dién-3-one
4-({4-[(11β,17β)-17-hydroxy-3-oxo-17-(pentafluoroéthyl)estra-4,9-dién-11-yl]benzyl}amino)benzamide
(11β,17β)-17-hydroxy-11-(4-{[méthyl(2-phényléthyl)-amino]méthyl}phényl)-17-(pentafluoroéthyl)estra-4,9-dién-3-one
(11β,17β)-17-hydroxy-17-(pentafluoroéthyl)-11-(4-{[4-(2-phényléthyl)pipérazin-l-yl]méthyl}phényl)estra-4,9-dién-3-one
(11β,17β)-17-hydroxy-17-(pentafluoroéthyl)-11-(4-{[4-(pyrazin-2-yl)pipérazin-1-yl]méthyl}phényl)estra-4,9-dién-3-one
(11β,17β)-17-hydroxy-17-(pentafluoroéthyl)-11-[4-({4-[2-(pyrrolidin-1-yl)éthyl]pipérazin-1-yl}méthyl)-phényl]estra-4,9-dién-3-one
(11β,17β)-17-hydroxy-17-(pentafluoroéthyl)-11-(4-{[4-(pyridin-4-ylméthyl)pipérazin-1-yl]méthyl}phényl)estra-4,9-dién-3-one
N-[4-({4-[(11β,17β)-17-hydroxy-3-oxo-17-(pentafluoroéthyl)estra-4,9-dién-11-yl]benzyl}amino)phényl]-acétamide
3-({4-[(11β,17β)-17-hydroxy-3-oxo-17-(pentafluoroéthyl)estra-4,9-dién-11-yl]benzyl}amino)benzoate de méthyle
3-({4-[(11β,17β)-17-hydroxy-3-oxo-17-(pentafluoroéthyl)estra-4,9-dién-11-yl]benzyl}amino)benzènesulfonamide
(11β,17β)-17-hydroxy-17-(pentafluoroéthyl)-11-{4-[(4-phénylpipéridin-1-yl)méthyl]phényl}estra-4,9-dién-3-one
4-{4-[(11β,17β)-17-hydroxy-3-oxo-17-(pentafluoroéthyl)-estra-4,9-dién-11-yl]benzyl}-N,N-diméthylpipérazine-1-carboxamide
(11β,17β)-17-hydroxy-17-(pentafluoroéthyl)-11-(4-{[4-(pyridin-2-ylméthyl)pipérazin-1-yl]méthyl}phényl)estra-4,9-dién-3-one
(11β,17β)-17-hydroxy-17-(pentafluoroéthyl)-11-(4-{[4-(pyridin-3-ylméthyl)pipérazin-1-yl]méthyl}phényl)estra-4,9-dién-3-one
2-(4-{4-[(11β,17β)-17-hydroxy-3-oxo-17-(pentafluoroéthyl)estra-4,9-dién-11-yl]benzyl}pipérazin-1-yl)-N-méthylacétamide
(11β,17β)-17-hydroxy-17-(pentafluoroéthyl)-11-[4-(pyrrolidin-1-ylméthyl)phényl]estra-4,9-dién-3-one
(11β,17β)-17-hydroxy-17-(pentafluoroéthyl)-11-(4-{[(pyridin-4-ylméthyl)amino]méthyl}phényl)estra-4,9-dién-3-one
(11β,17β)-17-hydroxy-11-(4-{[méthyl(phényl)amino]-méthyl}phényl)-17-(pentafluoroéthyl)estra-4,9-dién-3-one
2-({4-[(11β,17β)-17-hydroxy-3-oxo-17-(pentafluoroéthyl)estra-4,9-dién-11-yl}benzyl}amino)benzamide
(11β,17β)-17-hydroxy-17-(pentafluoroéthyl)-11-(4-{[4-(pyridin-4-yl)pipérazin-1-yl]méthyl}phényl)estra-4,9-dién-3-one
(11β,17β)-11-[4-({4-[2-(diméthylamino)éthyl]pipérazin-1-yl}méthyl)phényl]-17-hydroxy-17-(pentafluoroéthyl)-estra-4,9-dién-3-one
(11β,17β)-17-hydroxy-11-[4-({méthyl[2-(pyridin-2-yl)éthyl]amino}méthyl)phényl]-17-(pentafluoroéthyl)-estra-4,9-dién-3-one
3-({4-[(11β,17β)-17-hydroxy-3-oxo-17-(pentafluoroéthyl)estra-4,9-dién-11-yl]benzyl}amino)benzamide
4-({4-[(11β,17β)-17-hydroxy-3-oxo-17-(pentafluoroéthyl)estra-4,9-dién-11-yl]benzyl}amino)-N-phénylbenzamide
et leurs sels, produits de solvatation ou produits de solvatation des sels, y compris toutes les formes cristallines.

10. Composé selon l'une quelconque des revendications précédentes, destiné au traitement et à la prophylaxie de maladies.

11. Composé selon l'une quelconque des revendications 1 à 9, destiné au traitement et à la prophylaxie de fibroïdes utérins, de l'endométriose, de flux menstruels forts, de méningiomes, de carcinomes mammaires hormonodépendants et de troubles liés à la ménopause, ou à la contraception et la contraception d'urgence.

12. Utilisation d'un composé selon l'une quelconque des revendications 1 à 9, pour la fabrication d'un médicament destiné au traitement et/ou à la prophylaxie de maladies.

13. Utilisation d'un composé selon l'une quelconque des revendications 1 à 9, pour la fabrication d'un médicament destiné au traitement et/ou à la prophylaxie de fibroïdes utérins, de l'endométriose, de flux menstruels forts, de méningiomes, de carcinomes mammaires hormonodépendants et de troubles liés à la ménopause, ou à la contraception et la contraception d'urgence.

14. Médicament contenant un composé tel que défini dans l'une quelconque des revendications 1 à 9, en association avec une autre substance active.

15. Médicament selon la revendication 14, en association avec un adjuvant inerte, non toxique, pharmaceutiquement convenable.

16. Médicament selon la revendication 15, destiné au traitement et/ou à la prophylaxie de fibroïdes utérins, de l'endométriose, de flux menstruels forts, de méningiomes, de carcinomes mammaires hormonodépendants et de troubles liés à la ménopause, ou à la contraception et la contraception d'urgence.
